(19) Europäisches Patentamt
European Patent Office
Office européen des brevets

(11) EP 2 439 195 B1

(12) **EUROPEAN PATENT SPECIFICATION**

(45) Date of publication and mention of the grant of the patent:
**16.07.2014 Bulletin 2014/29**

(21) Application number: **10782884.0**

(22) Date of filing: **05.03.2010**

(51) Int Cl.:
*C07D 215/22* (2006.01) *C07D 215/233* (2006.01)
*C07D 239/88* (2006.01) *C07D 401/12* (2006.01)
*A61K 31/438* (2006.01) *A61K 31/444* (2006.01)
*A61K 31/517* (2006.01) *A61P 3/00* (2006.01)
*A61P 9/00* (2006.01) *A61P 11/00* (2006.01)
*A61P 25/00* (2006.01) *A61P 29/00* (2006.01)
*A61P 35/00* (2006.01) *A61P 37/08* (2006.01)

(86) International application number:
**PCT/CN2010/000272**

(87) International publication number:
**WO 2010/139180 (09.12.2010 Gazette 2010/49)**

(54) **NAPHTHALENE CARBOXAMIDE DERIVATIVES AS INHIBITORS OF PROTEIN KINASE AND HISTONE DEACETYLASE, PREPARATION METHODS AND USES THEREOF**

NAPHTHALENCARBOXAMIDDERIVATIVE ALS HEMMER DER PROTEINKINASE UND HISTONDEACETYLASE, HERSTELLUNGSVERFAHREN UND VERWENDUNGEN DAMIT

DÉRIVÉS DE NAPHTALÈNE CARBOXAMIDE EN TANT QU'INHIBITEURS DE PROTÉINE KINASE ET D'HISTONE DÉSACÉTYLASE, PROCÉDÉS DE PRÉPARATION ET UTILISATIONS

(84) Designated Contracting States:
**AT BE BG CH CY CZ DE DK EE ES FI FR GB GR HR HU IE IS IT LI LT LU LV MC MK MT NL NO PL PT RO SE SI SK SM TR**

(30) Priority: **04.06.2009 CN 200910143978**

(43) Date of publication of application:
**11.04.2012 Bulletin 2012/15**

(73) Proprietor: **Shenzhen Chipscreen Biosciences, Ltd.**
**Shenzhen, Guangdong 518057 (CN)**

(72) Inventors:
- **LU, Xianping**
  **Guangdong 518057 (CN)**
- **LI, Zhibin**
  **Guangdong 518057 (CN)**
- **SHAN, Song**
  **Guangdong 518057 (CN)**
- **YU, Jindi**
  **Guangdong 518057 (CN)**
- **NING, Zhiqiang**
  **Guangdong 518057 (CN)**

(74) Representative: **Kling, Simone**
**Lavoix Munich**
**Bayerstrasse 83**
**80335 München (DE)**

(56) References cited:
**WO-A1-2005/021553 CN-A- 1 933 839**

- **HARMANGE, JEAN-CHRISTOPHE ET AL.: 'Naphthamides as novel and potent vascular endothelial growth factor receptor tyrosine kinase inhibitors: design, synthesis, and evaluation.' J. MED. CHEM. vol. 51, 2008, pages 1649 - 1667, XP002668409**
- **DU, JUAN ET AL.: 'Molecular modeling studies of vascular endothelial growth factor receptor tyrosine kinase inhibitors using QSAR and docking.' JOURNAL OF MOLECULAR GRAPHICS AND MODELLING vol. 27, 05 November 2008, pages 642 - 654, XP025815659**
- **SIMPLÍCIO ANA L ET AL: "Prodrugs for amines", MOLECULES, MOLECULAR DIVERSITY PRESERVATION INTERNATIONAL, BASEL, CH, vol. 13, no. 3, 1 January 2008 (2008-01-01), pages 519-547, XP002503564, ISSN: 1420-3049, DOI: 10.3390/MOLECULES13030519**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

Remarks:

The file contains technical information submitted after the application was filed and not included in this specification

**Description**

**FIELD OF INVENTION**

The present invention relates to naphthalene carboxamide (naphthamide) derivatives which have protein kinases inhibition activities and histone deacetylases inhibition activities, the preparation method thereof and clinical use of the same in treating diseases associated with abnormal protein kinase activities and abnormal histone deacetylase activities.

**BACKGROUND OF THE INVENTION**

Protein kinases are a family of enzymes that catalyze the phosphorylation of proteins, in particular the hydroxy group of specific tyrosine, serine and threonine residues in proteins. Protein kinases play a critical role in the regulation of a wide variety of cellular processes, including metabolism, cell proliferation, cell differentiation, cell survival, environment-host reaction, immune response, and angiogenesis. Many diseases are associated with abnormal cellular responses triggered by protein kinase regulation. These diseases include inflammatory diseases, autoimmune diseases, cancer, nervous system diseases and neurodegenerative diseases, cardiovascular diseases, metabolic diseases, allergies, asthma and hormone-related diseases (Tan, S-L.,2006, J. Immunol., 176: 2872-2879; Healy, A. ea al.,2006, J. Immunol., 177: 1886-1893; Salek-Ardakani, S. et al.,2005, J. Immunol., 175: 7635-7641; Kim, J. et al.,2004, J. Clin. Invest., 114: 823-827). Therefore, considerable effort has been made to identify protein kinase inhibitors that are effective as therapeutic agents against these diseases.

The protein kinases can be conventionally divided into two classes, the protein tyrosine kinases (PTKs) and the serine-threonine kinases (STKs).

The protein tyrosine kinases (PTKs) can be divided into two classes: the non-transmembrane tyrosine kinases and transmembrane growth factor receptor tyrosine kinases (RTKs). At present, at least nineteen distinct subfamilies of RTKs have been identified, such as the epidermal growth factor receptor (EGFR), the vascular endothelial growth factor receptor (VEGFR), the platelet derived growth factor receptor (PDGFR), and the fibroblast growth factor receptor (FGFR).

The epidermal growth factor receptor (EGFR) family comprises four transmembrane tyrosine kinase growth factor receptors: HER1, HER2, HER3 and HER4. Binding of a specific set of ligands to the receptor promotes EGFR dimerization and results in the receptors autophosphorylation on tyrosine residues (Arteaga, C-L.,2001, Curr. Opin. Oncol., 6: 491-498). Upon autophosphorylation of the receptor, several downstream signal transduction pathways of EGFR become activated. The EGFR signal transduction pathways have been implicated in neoplastic processes, including cell cycle progression, inhibition of apoptosis, tumor cell motility, invasion and metastasis. EGFR activation also stimulates vascular endothelial growth factor (VEGF), which is the primary inducer of angiogenesis (Petit, A-M. et al.,1997, Am. J. Pathol., 151: 1523-1530). In experimental models, de-regulation of the EGFR-mediated signal transduction pathways is associated with oncogenesis (Wikstrand, C-J. et al.,1998, J Natl Cancer Inst., 90: 799-800). Mutations leading to continuous activation of amplification and over expression of EGFR proteins are seen in many human tumors, including tumors of breast, lung, ovaries and kidney. These mutations are a determinant of tumor aggressiveness (Wikstrand, C-J. et al.,1998, J Natl Cancer Inst., 90: 799-800). EGFR over expression is frequently seen in non-small cell lung cancer (NSCLC). Activity of EGFR can be inhibited either by blocking the extracellular ligand binding domain with the use of anti-EGFR antibodies or by the use of small molecules that inhibit the EGFR tyrosine kinase, thus resulting in inhibition of downstream components of the EGFR pathway (Mendelsohn, J., 1997, Clin. Can. Res., 3: 2707-2707).

The vascular endothelial growth factor (VEGF) is secreted by almost all solid tumors and tumor associated stroma in response to hypoxia. It is highly specific for vascular endothelium and regulates both vascular proliferation and permeability. Excessive expression of VEGF levels correlate with increased microvascular density, cancer recurrence and decreased survival (Parikh, A-A., 2004;, Hematol. Oncol. Clin. N. Am., 18:951-971). There are 6 different ligands for the VEGF receptor, VEGF-A through -E and placenta growth factor. Ligands bind to specific receptors on endothelial cells, mostly VEGFR-2. The binding of VEGF-A to VEGFR-1 induces endothelial cell migration. Binding to VEGFR-2 induces endothelial cell proliferation, permeability and survival. VEGFR-3 is thought to mediate lymphangiogenesis. The binding of VEGF to VEGFR-2 receptors results in activation and autophosphorylation of intracellular tyrosine kinase domains which further triggers other intracellular signaling cascades (Parikh, A-A., 2004, Hematol. Oncol. Clin. N. Am., 18:951-971).

The serine-threonine kinases (STKs) are predominantly intracellular although there are a few receptor kinases of the STK type. STKs are the most common forms of the cytosolic kinases that perform their function in the part of the cytoplasm other than the cytoplasmic organelles and cytoskelton.

Glycogen synthase kinase-3 (GSK-3) is a serine-threonine protein kinase comprised of $\alpha$ and $\beta$ isoforms that are each encoded by distinct genes. GSK-3 has been found to phosphorylate and modulate the activity of a number of

regulatory proteins. GSK-3 has been implicated in various diseases including diabetes, Alzheimer's disease, CNS disorders such as manic depressive disorder and neurodegenerative diseases, and cardiomyocyte hypertrophy (Haq, et al., 2000, J. Cell Biol., 151: 117).

Aurora-2 is a serine-threonine protein kinase that has been implicated in human cancer, such as colon cancer, breast cancer, and other solid tumors. This kinase is believed to be involved in protein phosphorylation that regulate cell cycle. Specifically, Aurora-2 may play a role in controlling the accurate segregation of chromosomes during mitosis. Misregulation of the cell cycle can lead to cellular proliferation and other abnormalities. In human colon cancer tissue, the Aurora-2 protein has been found to be over expressed (Schumacher, et al., 1998, J. Cell Biol., 143: 1635-1646; Kimura et al., 1997, J. Biol. Chem., 272: 13766-13771).

The cyclin-dependent kinases (CDKs) are serine-threonine protein kinase that regulates mammalian cell division. To date, nine kinase subunits (CDK 1-9) have been identified. Each kinase associates with a specific regulatory partner and together makes up the active catalytic moiety. Uncontrolled proliferation is a hallmark of cancer cells, and misregulation of CDK function occurs with high frequency In many important solid tumors. CDK2 and CDK4 are of particular interest because their activities are frequently misregulated in a wide variety of human cancers.

Raf kinase, a downstream effector of *ras* oncoprotein, is a key mediator of signal-transduction pathways from cell surface to the cell nucleus. Inhibition of raf kinase has been correlated *in vitro* and *in vivo* with inhibition of the growth of variety of human tumor types (Monia et al., 1996, Nat. Med., 2: 668-675).

Other serine-threonine protein kinases include the protein kinase A, B and C. These kinases, known as PKA, PKB and PKC, play key roles in signal transduction pathways.

Many attempts have been made to identify small molecules which act as protein kinase inhibitors useful in the treatment of diseases associated with abnormal protein kinase activities. For example, cyclic compounds (U.S. Pat. No. 7,151,096), bicyclic compounds (U.S. Pat. No. 7,189,721), tricyclic compounds (U.S. Pat. No. 7,132,533), (2-oxindol-3-methylidene) acetic acid derivatives (U.S. Pat. No. 7,214,700), 3-(4-amidopyrrol-2-ylmethylidene)-2-indolinone derivatives (U.S. Pat. No. 7,179,910), fused pyrazole derivatives (U.S. Pat. No. 7,166,597), aminofurazan compounds (U.S. Pat. No. 7,157,476), pyrrole substituted 2-indolinone compounds (U.S. Pat. No. 7,125,905), triazole compounds (U.S. Pat. No. 7,115,739), pyrazolylamine substituted quinazoline compounds (U.S. Pat. No. 7,098,330) and indazole compounds (U.S. Pat. No. 7,041,687) have all been described as protein kinase inhibitors.

CN 1933839A and WO 2005/070891 disclose compounds of formula (a) for treating diseases such as inflammation, cancer, cardiovascular conditions and diseases associated with protein kinases. $R^1$ is selected from monocyclic heteroaryl or bicyclic heteroaryl; Ring A is selected from naphthalene ring, bicyclic heteroaryl, etc.; X is selected from O, S, NH, etc.; Y is selected from -NH-, -NHC(O)-, -C(O)NH-, etc.; R is selected from aryl, heterocyclyl. cycloalkyl, alkyl, etc. These compounds showed inhibition of VEGFR (< 10μM).

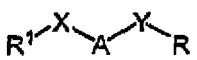

(a)

WO 2005/021553A1 discloses naphthalene carboxamides of formula (b) capable of modulating the activity of receptor kinases such as VEGFR and PDGFR, $X^1$ is selected from O or S; Each of $R^{1a}$, $R^{1b}$, $R^{1c}$, $R^{1d}$ and $R^{1e}$ is independently selected from H, halogen, OH, etc.; R3 is selected from H, aryl, heterocyclyl, ect.; One of $R^{2a}$ and $R^{2b}$ is -C(O)NHR$^4$, and $R^4$ is selected from H, alkyl, aryl, heterocyclyl, ect. Some compounds showed high inhibition of VEGFR ($IC_{50}$<10nM).

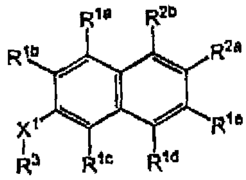

(b)

Several protein kinase inhibitors such as Glivec, Suten, and Sorafenib have been successfully approved by FDA for anti-cancer therapy. Their clinic uses demonstrated clear advantages over existing chemotherapeutical treatments, fueling continuing interests in innovation of mechanism-based treatments and improvement of chemical scaffolds to discover new compounds with excellent oral bioavailability, better anti-tumor activity, and lower toxicity.

## SUMMARY OF THE INVENTION

One purpose of the present invention is to provide certain naphthamide derivatives which are capable of selectively inhibiting protein kinases and histone deacetylases.
Another purpose of the present invention is to provide the preparation methods of said compounds.
A further purpose of the present invention is to provide clinical use of said compounds in treating diseases related to abnormal protein kinase activities and abnormal histone deacetylase activities.;

## BRIEF DESCRIPTION OF THE DRAWINGS

Figure 1 graphically illustrates the antitumor activity of compound 31 on nude mouse tumor transplanted from human A549 lung cancer, wherein Vehicle represents carrier, Sutent represents existing drug sunitinib, and comp 31 represents compound 31.
Figure 2 graphically illustrates the antitumor activity of compound 31 on nude mouse tumor transplanted from human HCT-8 colon cancer, wherein Vehicle represents carrier, Sutent represents existing drug sunitinib, and comp 31 represents compound 31.
Figure 3 graphically illustrates the antitumor activity of compound 31 on nude mouse tumor transplanted from human SSMC7721 liver cancer, wherein Vehicle represents carrier, Sutent represents existing drug sunitinib, and comp 31 represents compound 31.
Figure 4 graphically illustrates the antitumor activity of compound 33 and compound 34 on nude mouse tumor transplanted from human HCT-8 colon cancer, wherein Vehicle represents carrier, Sutent represents existing drug sunitinib, comp 33 represents compound 33, and com 34 represents compound 34.
Figure 5 graphically illustrates the antitumor activity of compound 33 and compound 37 on nude mouse tumor transplanted from human HCT-8 colon cancer, wherein Vehicle represents carrier, Sutent represents existing drug sunitinib, comp 33 represents compound 33, and com 37 represents compound 37.
Figure 6 graphically illustrates the antitumor activity of compound 33 and compound 37 on nude mouse tumor transplanted from human SSMC7721 liver cancer, wherein Vehicle represents carrier, Sutent represents existing drug sunitinib, comp 33 represents compound 33, and com 37 represents compound 37.

## DETAILED DESCRIPTION OF THE INVENTION

Histone deacetylase (HDAC) proteins play a critical role in regulating gene expression *in vivo* by altering the accessibility of transcription factors to genomic DNA . Specifically, HDAC proteins remove the acetyl group of acetyllysine residues on histones, which can result in nucleosomal remodelling (Grunstein, M., 1997, Nature, 389: 349-352). Due to their governing role in gene expression, HDAC proteins are associated with a variety of cellular events, including cell cycle regulation, cell proliferation, differentiation, reprogramming of gene expression, and cancer development (Ruijter, A-J-M., 2003, Biochem. J., 370: 737-749; Grignani, F., 1998, Nature, 391: 815-818; Lin, R-J.,1998, 391: 811-814; Marks, P-A., 2001, Nature Reviews Cancer, 1: 194). Abnormal deacetylation caused by misregulation of histone deacetylation has been implicated in various diseases, such as Rubinstein-Taybi syndrome, fragile X syndrome, neurodegenerative diseases, cardiovascular and metabolic disease, rheumatoid disease, leukemia and other kinds of cancer(Langley B et al., 2005, Current Drug Targets-CNS & Neurological Disorders, 4: 41-50). HDAC inhibitors have been demonstrated through experiments to reduce the growth of tumor in human and animals, including lung cancer, stomach cancer, breast cancer, prostrate cancer, lymphoma and the like (Dokmanovic, M.,2005, J. Cell Biochenm., 96: 293-304).
Mammalian HDACs can be divided into three classes according to sequence homology. Class I consists of the yeast Rpd3-like proteins (HDAC 1, 2, 3, 8 and 11). Class II consists of the yeast HDA1-like proteins (HDAC 4, 5, 6, 7, 9 and 10). Class III consists of the yeast SIR2-like proteins (SIRT 1, 2, 3, 4, 5, 6 and 7).
The activity of HDAC1 has been linked to cell proliferation, a hallmark of cancer. Particularly, mammalian cells with knock down of HDAC1 expression using siRNA were antiproliferative (Glaser, K-B., 2003, Biochem. Biophys. Res. Comm., 310: 529-536). While the knock out mouse of HDAC1 was embryonic lethal, the resulting stem cells displayed altered cell growth (Lagger, G., 2002, EMBO J., 21: 2672-2681). Mouse cells overexpressing HDAC1 demonstrated a lengthening of $G_2$ and M phases and reduced growth rate (Bartl. S., 1997, Mol. Cell Biol., 17: 5033-5043). Therefore, the reported data implicate HDAC1 involves in cell cycle regulation and cell proliferation.
HDAC2 regulates expression of many fetal myocardial protein isoforms. HDAC2 deficiency or chemical inhibition of histone deacetylase may prevent the re-expression of fetal genes and attenuate cardiac hypertrophy. Resistance to hypertrophy was associated with increased expression of the gene encoding inositol polyphosphate-5-phosphatase f (Inpp5f) resulting in activation of glycogen synthase kinase 3β (Gsk3β) via inactivation of thymoma proto-oncogene (Akt)

and 3-phosphoinositide-dependent protein kinase-1 (Pdk1). In contrast, HDAC2 transgenic mice had augmented hypertrophy associated with inactivated Gsk3β. Chemical inhibition of activated Gsk3β allowed HDAC2-deficient adults to become sensitive to hypertrophic stimulation. These results suggest that HDAC2 is an important molecular target of HDAC inhibitors in the heart and that HDAC2 and Gsk3β are both components of a regulatory pathway providing an attractive therapeutic target for the treatment of cardiac hypertrophy and heart failure (Trivedi, C-M., 2007, Nat. Med,. 13: 324-331).

HDAC3 are maximally expressed in proliferating crypt cells in normal intestine. Silencing of HDAC3 expression in colon cancer cell lines resulted in cell growth inhibition, a decrease in cell survival, and increased apoptosis. Similar results were observed for HDAC2 and, to a lesser extent, for HDAC1. HDAC3 gene silencing also selectively induced expression of alkaline phosphatase, a marker of colon cell maturation. Overexpression of HDAC3 inhibited basal and butyrate-induced p21 transcription, whereas silencing of HDAC3 stimulated p21 promoter activity and expression. These findings identify HDAC3 as a gene deregulated in human colon cancer and as a novel regulator of colon cell maturation and p21 expression (Wilson, A-J., 2006, J. Biol. Chem., 281: 13548-13558).

HDAC6 is a subtype of the HDAC family that deacetylates alpha-tubulin and increases cell motility. Using quantitative real-time reverse transcription polymerase chain reaction and Western blots on nine oral squamous cell carcinoma (OSCC)-derived cell lines and normal oral keratinocytes (NOKs), HDAC6 mRNA and protein expression were commonly up-regulated in all cell lines compared with the NOKs. Immunofluorescence analysis detected HDAC6 protein in the cytoplasm of OSCC cell lines. Similar to OSCC cell lines, HDAC6 up-regulation were evident in both mRNA (74%) and protein (51%) levels of primary human OSCC tumors. Among the clinical variables analyzed, the clinical tumor stage was found to be associated with the HDAC6 expression states. The analysis indicated a significant difference in the HDAC6 expression level between the early stage (stage I and II) and advanced-stage (stage III and IV) tumors (P=0.014). These results suggest that HDAC6 expression may be correlated with tumor aggressiveness and offer clues to the planning of new treatments (Sakuma, T., 2006, Int. J. Oncol., 29: 117-124).

Epigenetic silencing of functional chromosomes by HDAC is one of major mechanisms occurred in many pathological processes, in which function-related genes are repressed or reprogrammed by HDAC activities leading to the loss of phenotypes in terminal differentiation, maturation and growth control, and the loss of functionality of tissues. For example, tumor suppressor genes are often silenced during development of cancer and inhibitor of HDAC can depress the expression of these tumor suppressor genes, leading to inhibition of cell growth and differentiation (Glaros S et al., 2007, Oncogene June 4 Epub ahead of print; Mai, A, et al., 2007, Int J. Biochem Cell Bio., April 4, Epub ahead of print; Vincent A. et al., 2007, Oncogene, April 30, Epub ahead of print; our unpublished results).Repression of structural genes such as FXN in Friedreich's ataxia and SMN in spinal muscular atrophy can be reversed by HDAC inhibitors that leads to re-expression of FXN and SMN genes and resume the functions in the tissues (Herman D et al., 2006, Nature Chemical Biology, 2(10):551-8; Avila AM et al., 2007, J Clinic Investigation, 117(3)659-71; de Bore J, 2006, Tissue Eng. 12(10):2927-37). Induction of entire MHC II family gene expression through reprogramming of HDAC "hot spot" in chromosome 6p21-22 by HDAC inhibitor further extends epigenetic modulation of immune recognition and immune response (Gialitakis M et al., 2007, Nucleic Acids Res., 34(1);765-72).

Several classes of HDAC inhibitors have been identified, including (1) short-chain fatty acids, e.g. butyrate and phenylbutyrate; (2) organic hydroxamic acids, e.g. suberoylanilide hydroxamic acid (SAHA) and trichostatin A (TSA); (3) cyclic tetrapeptides containing a 2-amino-8-oxo-9,10-expoxydecanoyl (AOE) moiety, e.g. trapoxin and HC-toxin; (4) cyclic tetrapeptides without the AOE moiety, e.g. apicidin and FK228; and (5) benzamides, e.g. MS-275 (EP0847992A1, US2002/0103192A1, WO02/26696A1, WO01/70675A2, WO01/18171A2). Although, HDAC inherited very promising biological roles as a drug target, the success of SAHA from Merck is currently only limited to the treatment of cutaneous T cell lymphoma whereas no major solid tumors yet been reported to be highly effective by this treatment. Therefore, there is still a need to discover new compounds with stronger HDAC inhibitory activity and anti-cancer activity, more selective inhibition on different subtype of HDAC, and lower toxicity.

The favorite metaphor for cancer drug developers has long been the target therapy. One hoped to design a drug that could hit tumor cells in one specific target, killing tumor cells while leaving normal cells undamaged. Cancer cells, however, can use multiple biological triggers and pathways to grow and spread throughout the body. Hitting them in one target will also render them to regroup and redeploy along new growth paths. That realization has led to the development of combination target therapies, which are becoming the new paradigm for cancer treatment. Several multi-target kinase inhibitors are now in development, two of them, Sorafenib and Suten, are already approved in the United States. For example, Sorafenib, developed by Bayer Pharmaceuticals, is the first drug targeting both the RAF/MEK/ERK pathway (involved in cell proliferation) and the VEGFR2/PDGFRβ signaling cascade (involved in angiogenesis). This drug was first approved in December 2005 for advanced kidney cancer. However, these target therapies, although are effective against some solid tumors, but far from satisfaction in terms of reaching a better efficacy and while maintaining acceptable side-effects associated with the treatments against other solid tumors.

Provided herein are new chemical compounds that combine anti-angiogenesis and anti-proliferation activities of RTK inhibitors together with differentiation-inducing, immune modulation, cell cycle arrest and apoptosis-induction

activities of HDAC inhibitors, to reach a better efficacy against solid tumors while overcoming side effects such as hypertension, QT prolongation, thyroid gland regression, skin rash and discoloration, and pains associated with currently marketed RTK inhibitors.

Particularly, the present invention provides a compound having the structure represented by formula (I):

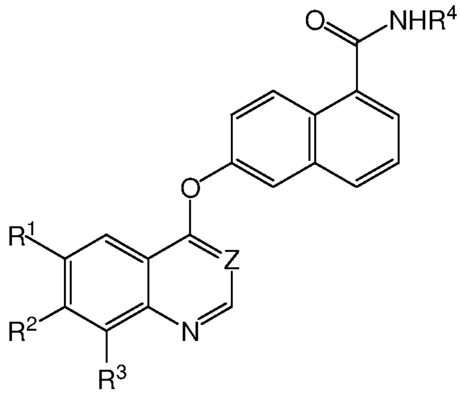

(I)

including its free form, salt form, enantiomer, diastereomer or hydrate, wherein

Z is CH or N;
$R^1$, $R^2$ and $R^3$ are each hydrogen, halo, alkyl, alkoxy or trifluoromethyl;
$R^4$ is

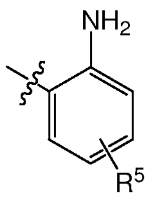

, or

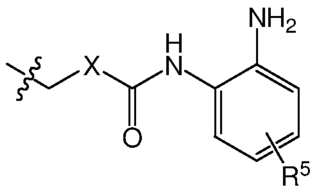

;

X is a benzene ring or a pyridine ring;
$R^5$ is one or more substituents selected from the group consisting of hydrogen, halo, alkyl, alkoxy and trifluoromethyl.

In the preferred embodiment, the compounds of this invention are those of the formula (I), wherein
Z is CH;
$R^1$, $R^2$ and $R^3$ are each hydrogen, halo, alkyl, alkoxy or trifluoromethyl;
$R^4$ is

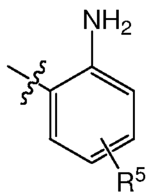

, or

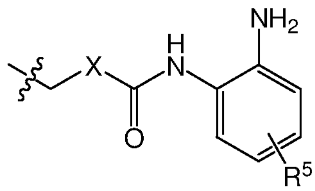

;

X is a benzene ring or a pyridine ring;
$R^5$ is one or more substituents selected from the group consisting of hydrogen, halo, alkyl, alkoxy and trifluoromethyl.

In another preferred embodiment, the compounds of this invention are those of the formula (I), wherein
Z is CH;
$R^1$, $R^2$ and $R^3$ are each hydrogen or alkoxy;
$R^4$ is

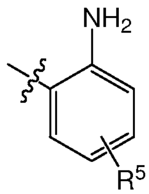
, or
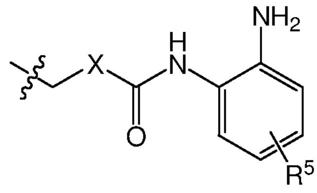
;

X is a benzene ring or a pyridine ring;
$R^5$ is one or more substituents selected from the group consisting of hydrogen, halo, alkyl, alkoxy and trifluoromethyl.

In another more preferred embodiment, the compounds of this invention are those of the formula (I), wherein
Z is CH;
$R^1$ and $R^2$ are each hydrogen or methoxy;
$R^3$ is H;
$R^4$ is

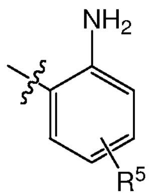
, or
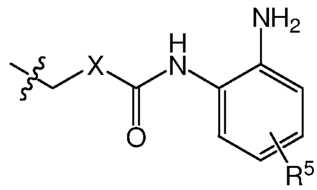
;

X is a benzene ring or a pyridine ring;
$R^5$ is one or more substituents selected from the group consisting of hydrogen, halo, alkyl, alkoxy and trifluoromethyl.

In another most preferred embodiment, the compounds of this invention are those of the formula (I), wherein
Z is CH;
$R^1$ and $R^2$ are each hydrogen or methoxy;
$R^3$ is H;
$R^4$ is

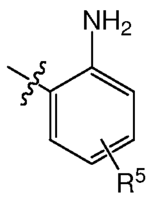
, or
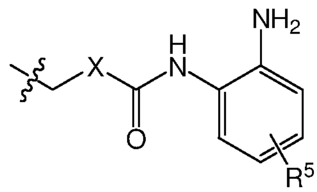
;

X is a benzene ring or a pyridine ring;
$R^5$ is H or F.

The term "halo" as used herein means fluorine, chlorine, bromine or iodine.
The term "alkyl" as used herein includes linear, branched or cyclic alkyls, for example, methyl, ethyl, n-propyl, iso-propyl, n-butyl, iso-butyl, tert-butyl, n-pentyl, iso-pentyl, n-hexyl, iso-hexyl, cyclopropyl, cyclobutyl, cyclopentyl, cyclohexyl and the like.

The term "alkoxy" as used herein means a group formed by attachment of an alkyl radical with an oxygen atom, wherein the oxygen atom has the ability of free bonding. Examples thereof include methoxy, ethoxy, propoxy, butoxy, pentoxy, isopropoxy, tert-butoxy, cyclopropoxy, cyclohexyloxy and the like.

The compounds of this invention can be prepared as follows:

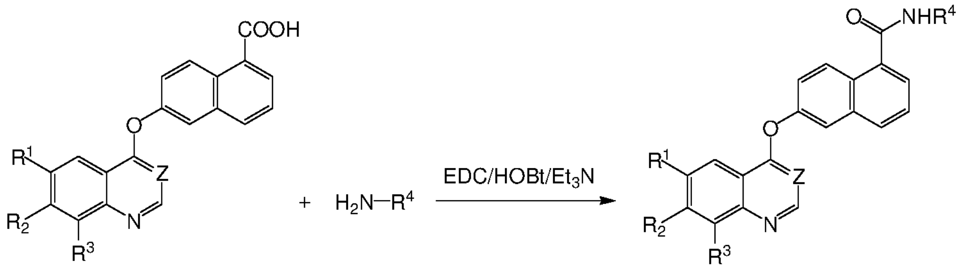


( **II** ) ( **III** ) ( **I** )

The compound of formula (**II**) is condensed with a compound of formula (**III**) to give the title compound (**I**). The condensation reaction is conducted by using a peptide condensing agent as a catalyst, such as 1-ethyl-3-(3-dimethylaminopropyl)carbodiimide (EDC), N,N'-dicyclohexylcarbodiimide (DCC), N,N'-carbonyldiimidazole (CDI), etc. The reaction may be conducted at 0 to 80°C for 4 to 72 hours. Solvents which may be used are normal solvents such as benzene, toluene, tetrahydrofuran, dioxane, dichloromethane, chloroform, N, N-dimethylformamide, etc. If necessary, a base such as sodium hydroxide, triethylamine or pyridine may be added to the reaction system.

The compounds of formula (**II**) can be prepared as follows:

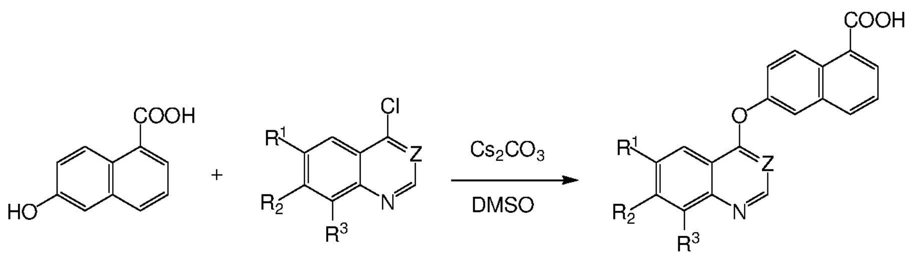


( **IV** ) ( **II** )

Commercially available 6-hydroxynaphthoic acid is heated in the presence of cesium carbonate and the appropriately substituted 4-chloroquinoline (**IV**) in DMSO to provide naphthoic acids (**II**). The reaction may be conducted at 130 to 140 °C for 3 to 24 hours.

The compounds of formula (**III**) are commercially available or prepared as follows:

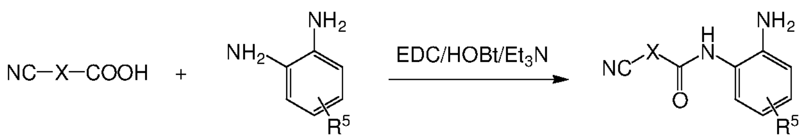


( **V** ) ( **VI** ) ( **VII** )

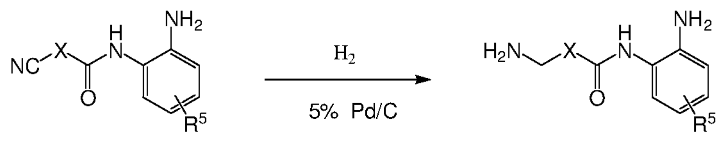


( **VII** ) ( **IIIa** )

Commercially available compound (**V**) is condensed with a commercially available compound (**VI**) to provide compound (**VII**). The condensation reaction is conducted by using a peptide condensing agent as a catalyst, such as 1-ethyl-3-(3-dimethylaminopropyl)carbodiimide (EDC), N,N'-dicyclohexylcarbodiimide (DCC), N,N'-carbonyldiimidazole (CDI), etc. The reaction may be conducted at 0 to 60°C for 2 to 72 hours. Solvents which may be used are normal solvents such as benzene, toluene, tetrahydrofuran, dioxane, dichloromethane, chloroform, N,N-dimethylformamide, etc. If necessary, a base such as sodium hydroxide, triethylamine or pyridine may be added to the reaction system.

The compound (**VII**) is dissolved in methanol and hydrogenated using 5% palladium on charcoal as a catalyst to yield compound (**IIIa**). The reaction may be conducted at room temperature. If necessary, an acid such as sulfuric acid may be added to the reaction system.

The compounds represented by formula (**I**) may be purified by the conventional separation methods such as extraction, recrystallization, column chromatography and the like.

The compounds represented by formula (**I**) are capable of inhibiting protein kinases and histone deacetylases simultaneously and are therefore useful in treating diseases associated with abnormal protein kinase activities and abnormal histone deacetylase activities. In particular, they are highly effective against hematological malignancy and solid tumors.

The compounds represented by formula (**I**) may be formulated into common pharmaceutical preparations, such as tablets, capsules, powders, syrups, solutions, suspensions, injections, ointments, and the like. The preparations may contain a compound of formula (**I**) as an active ingredient, together with pharmaceutically acceptable carriers, excipients and diluents. Such preparation typically contains from 0.5 to 70%, preferably 1 to 20% by weight of activeingredient.
The pharmaceutically acceptable carriers, excipients and diluents herein include, but not limited to those listed in ≪ Handbook of Pharmaceutical Excipients ≫ (American Pharmaceutical Association, October, 1986).
The compounds represented by formula (**I**) herein may be clinically administered to mammals, including man, via oral or injection routes. Administration by the oral route is preferred. The dosage administered is in the range of 0.0001 to 200 mg/kg body weight per day, preferably in the range of 0.01 to 100 mg/kg body weight per day, and most preferably in the range of 0.1 to 50 mg/kg body weight per dayHowever, the optimal dosage varies with the individual subject being treated, generally smaller dose is administered initially and thereafter increments is made.

Representative compounds of the present invention are shown in Table 1 below. The compound numbers correspond to the "Example numbers" in the Examples section. That is, the synthesis of compound 1 as shown in the Table 1 is described in "Example 1" and the synthesis of compound 44 as shown in the Table 1 is described in "Example 44".

Table 1 representative compounds of the present invention

| Example | Structure | Name |
|---|---|---|
| 16 | 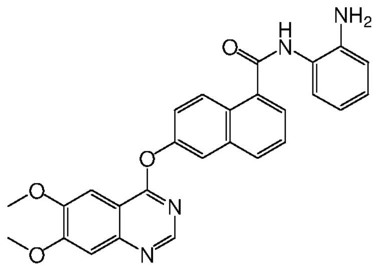 | N-(2-aminophenyl)-6-(6,7-dimethoxyquinazolin-4-yloxy)-1-naphthamide |
| 17 | 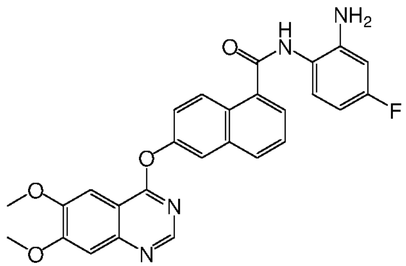 | N-(2-amino-4-fluorophenyl)-6-(6,7-dimethoxyquinazolin-4-yloxy)-1-naphthamide |
| 18 | 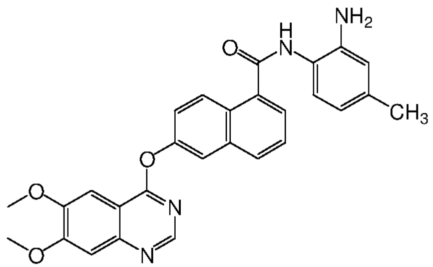 | N-(2-amino-4-methylphenyl)-6-(6,7-dimethoxyquinazolin-4-yloxy)-1-naphthamide |
| 19 | 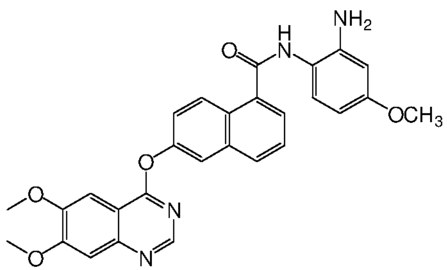 | N-(2-amino-4-methoxyphenyl)-6-(6,7-dimethoxyquinazolin-4-yloxy)-1-naphthamide |

(continued)

| Example | Structure | Name |
|---|---|---|
| 20 | 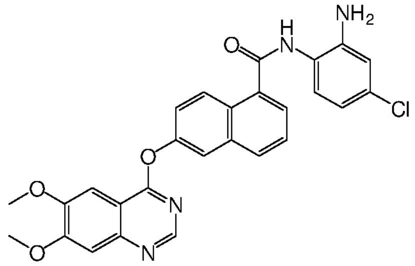 | N-(2-amino-4-chlorophenyl)-6-(6,7-dimethoxyquinazolin-4-yloxy)-1-naphthamide |
| 21 | 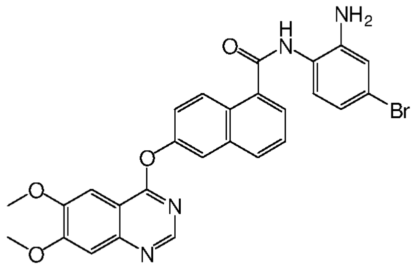 | N-(2-amino-4-bromophenyl)-6-(6,7-dimethoxyquinazolin-4-yloxy)-1-naphthamide |
| 22 | 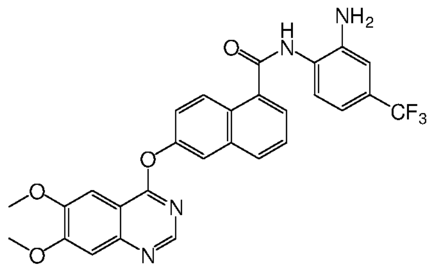 | N-(2-amino-4-trifluoromethyl-phenyl)-6-(6,7-dimethoxy-quinazolin-4-yloxy)-1-naphthamide |

(continued)

| Example | Structure | Name |
| --- | --- | --- |
| 23 | 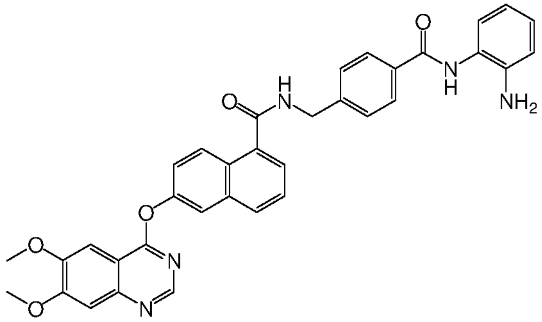 | N-(4-((2-aminophenyl)carbamoyl)-benzyl)-6-(6,7-dimethoxy-quinazolin-4-yloxy)-1-naphthamide |
| 24 | 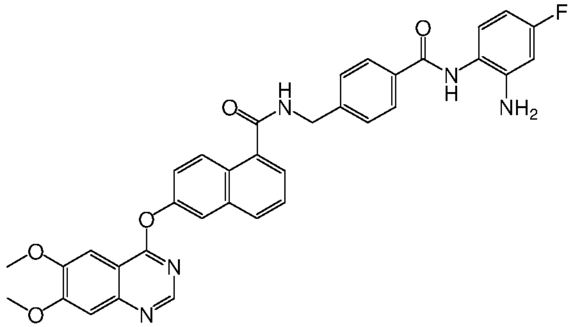 | N-(4-((2-amino-4-fluorophenyl)-carbamoyl)benzyl)-6-(6,7-dimethoxyquinazolin-4-yloxy)-1-naphthamide |
| 25 | 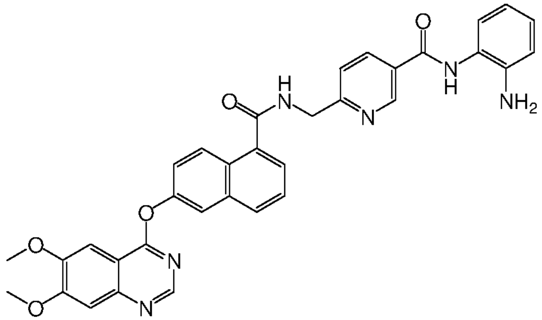 | N-(2-aminophenyl)-6-((2-(6,7-dimethoxyquinazolin-4-yloxy)-1-naphthamido)methyl)nicotinamide |

(continued)

| Example | Structure | Name |
|---|---|---|
| 26 | 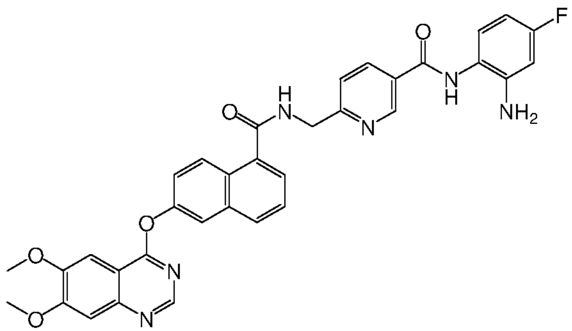 | N-(2-amino-4-fluorophenyl)-6-((2-(6,7-dimethoxyquinazolin-4-yloxy)-1-naphthamido)methyl)-nicotinamide |
| 27 | 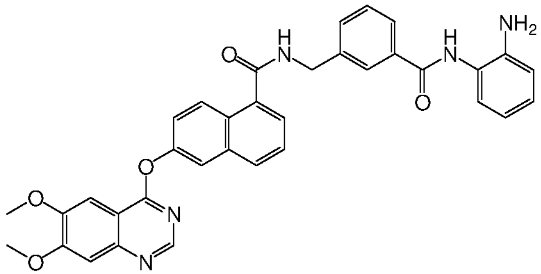 | N-(3-((2-aminophenyl)carbamoyl)-benzyl)-6-(6,7-dimethoxy-quinazolin-4-yloxy)-1-naphthamide |
| 28 | 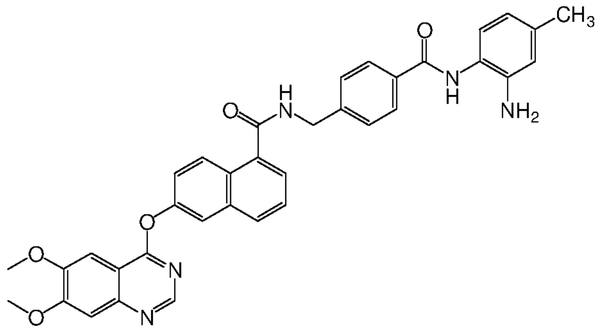 | N-(4-((2-amino-4-methylphenyl)-carbamoyl)benzyl)-6-(6,7-dimethoxyquinazolin-4-yloxy)-1-naphthamide |

(continued)

| Example | Structure | Name |
|---|---|---|
| 29 | 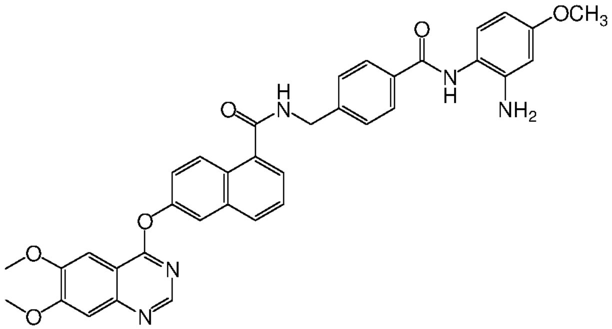 | N-(4-((2-amino-4-methoxyphenyl)-carbamoyl)benzyl)-6-(6,7-dimethoxyquinazolin-4-yloxy)-1-naphthamide |
| 30 | 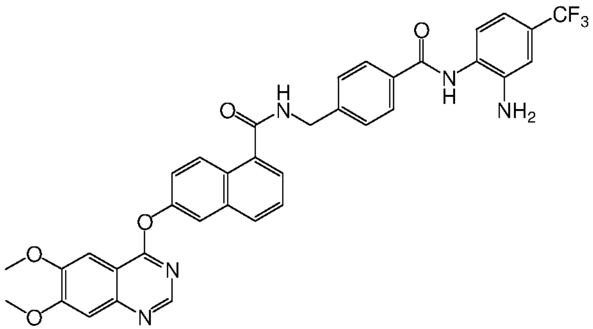 | N-(4-((2-amino-4-trifluoromethyl-phenyl)carbamoyl)benzyl)-6-(6,7-dimethoxyquinazolin-4-yloxy)-1-naphthamide |
| 31 | 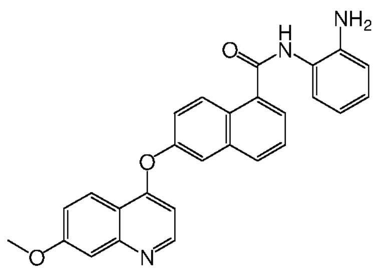 | N-(2-aminophenyl)-6-(7-methoxyquinolin-4-yloxy)-1-naphthamide |

(continued)

| Example | Structure | Name |
|---|---|---|
| 32 | 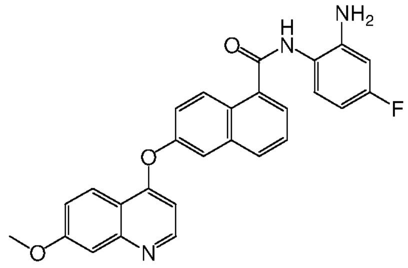 | N-(2-amino-4-fluorophenyl)-6-(7-methoxyquinolin-4-yloxy)-1-naphthamide |
| 33 | 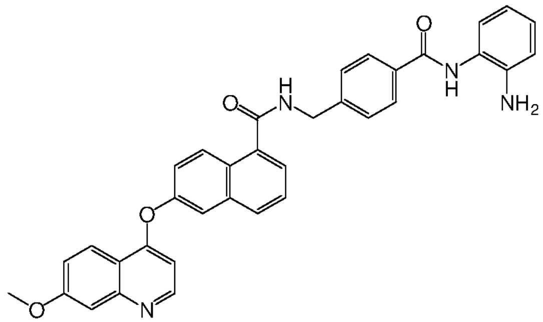 | N-(4-((2-aminophenyl)carbamoyl)-benzyl)-6-(7-methoxyquinolin-4-yloxy)-1-naphthamide |
| 34 | 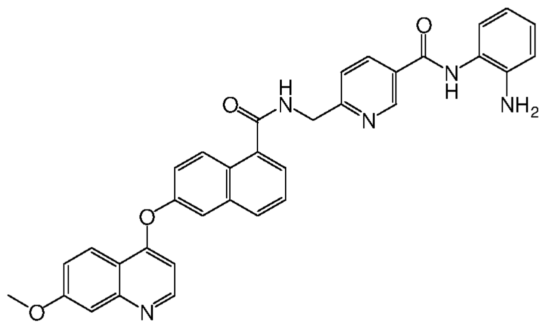 | N-(2-aminophenyl)-6-((2-(7-methoxyquinolin-4-yloxy)-1-naphthamido)methyl)nicotinamide |

(continued)

| Example | Structure | Name |
|---|---|---|
| 35 | 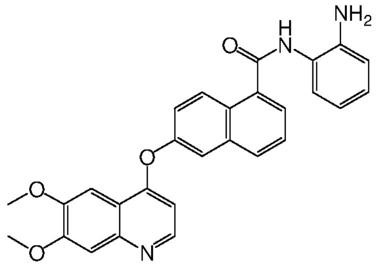 | N-(2-aminophenyl)-6-(6,7-dimethoxyquinolin-4-yloxy)-1-naphthamide |
| 36 | 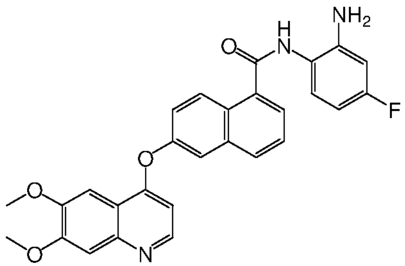 | N-(2-amino-4-fluorophenyl)-6-(6,7-dimethoxyquinolin-4-yloxy)-1-naphthamide |
| 37 | 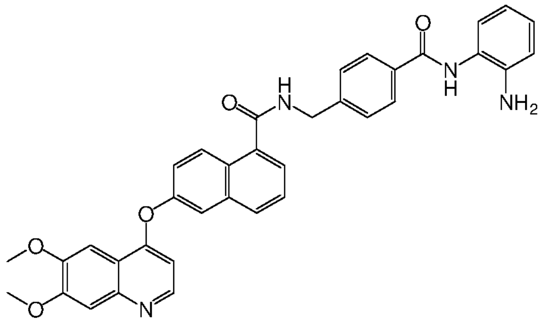 | N-(4-((2-aminophenyl)carbamoyl)-benzyl)-6-(6,7-dimethoxy-quinolin-4-yloxy)-1-naphthamide |

(continued)

| Example | Structure | Name |
|---|---|---|
| 38 | 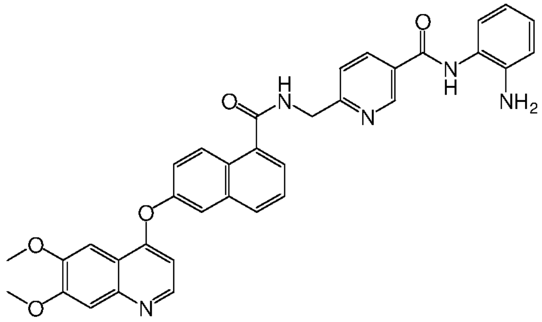 | N-(2-aminophenyl)-6-((2-(6,7-dimethoxyquinolin-4-yloxy)-1-naphthamido)methyl)nicotinamide |
| 39 | 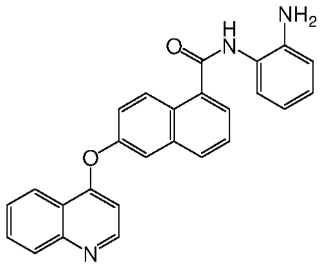 | N-(2-aminophenyl)-6-(quinolin-4-yloxy)-1-naphthamide |
| 40 | 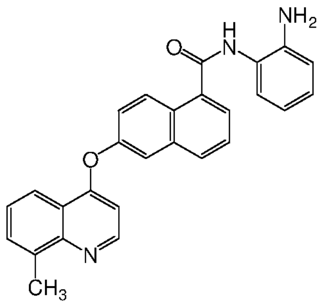 | N-(2-aminophenyl)-6-(8-methyl-quinolin-4-yloxy)-1-naphthamide |

(continued)

| Example | Structure | Name |
|---|---|---|
| 41 | 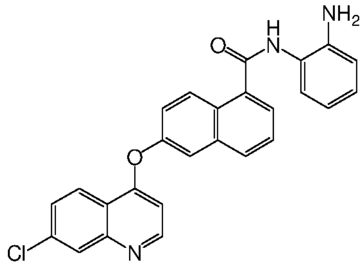 | N-(2-aminophenyl)-6-(7-chloro-quinolin-4-yloxy)-1-naphthamide |
| 42 | 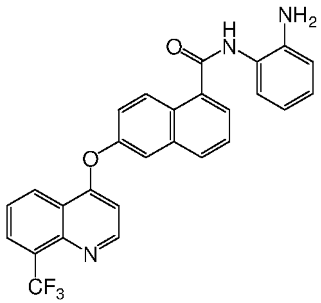 | N-(2-aminophenyl)-6-(8-(trifluoromethyl)quinolin-4-yloxy)-1-naphthamide |
| 43 | 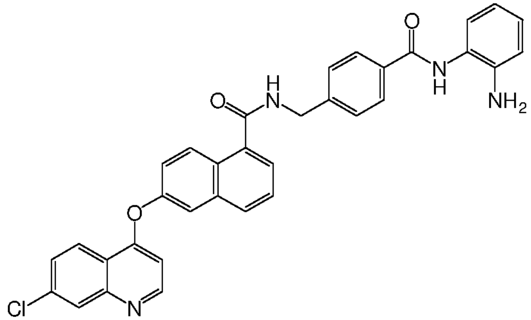 | N-(4-((2-aminophenyl)carbamoyl)-benzyl)-6-(7-chloroquinolin-4-yloxy)-1-naphthamide |

(continued)

| Example | Structure | Name |
|---|---|---|
| 44 | 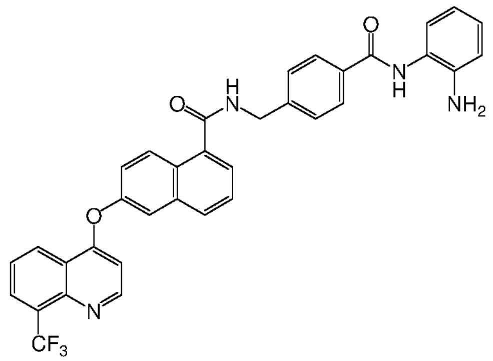 | N-(4-((2-aminophenyl)carbamoyl)-benzyl)-6-(8-(tritluoromethyl)-quinolin-4-yloxy)-1-naphthamide |

The present invention will be further illustrated in combination with examples below, however, the protection scope of the present invention is not limited to these examples. Further, the percentages described herein are by weight unless otherwise specified. Any range of numbers recited in the specification, such as units of measure, reaction conditions, physical states of a compound or percentages, is intended to provide reference literally and expressly. Those skilled in the art, when carrying out the present invention, using temperatures, concentrations, amounts, carbon numbers, and the like that fall outside of the range or different from a single value, will achieve the desired result.

Example 1

Preparation of 6-(6,7-dimethoxyquinazolin-4-yloxy)-1-naphthoic acid

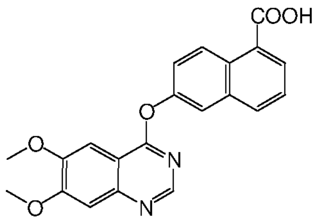

6-Hydroxy-1-naphthoic acid (1.43 g, 7.6 mmol) was dissolved in 38 ml of DMSO, then cesium carbonate (7.5 g, 22.9 mmol) and 4-chloro-6,7-dimethoxy-quinazoline (2.05 g, 9.14 mmol) were added. The mixture was heated at 140 °C for 3 hours. When the reaction was finished the mixture was cooled to room temperature and diluted with 40 mL of $H_2O$. The mixture was neutralized with 2 N HCl to pH=6.5. The deposited solids were filtered, washed with $H_2O$, dried and recrystallized from methanol to give the title compound (1.68 g, 59% yield) as a brown solid. LC-MS (m/z) 377 (M+1).

Example 2

Preparation of 6-(7-methoxyquinolin-4-yloxy)-1-naphthoic acid

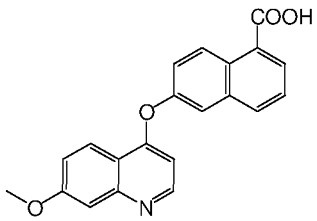

The title compound (1.73 g, 66% yield) was prepared as a brown solid from 6-hydroxy-1-naphthoic acid (1.43 g, 7.6 mmol) and 4-chloro-7-methoxyquinoline (1.77 g, 9.14 mmol) by an analogous procedure to that described in example 1. LC-MS (m/z) 346 (M+1).

Example 3

Preparation of 6-(6,7-dimethoxyquinolin-4-yloxy)-1-naphthoic acid

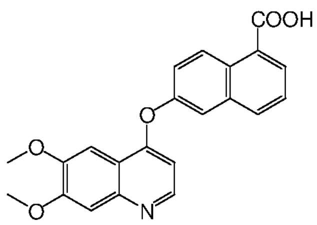

The title compound (1.95 g, 68% yield) was prepared as a brown solid from 6-hydroxy-1-naphthoic acid (1.43 g, 7.6 mmol) and 4-chloro-6,7-dimethoxyquinoline (2.04 g, 9.14 mmol) by an analogous procedure to that described in example 1. LC-MS (m/z) 376 (M+1).

Example 4

Preparation of 6-(quinolin-4-yloxy)-1-naphthoic acid

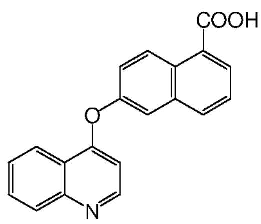

The title compound (1.24 g, 52% yield) was prepared as a brown solid from 6-hydroxy-1-naphthoic acid (1.43 g, 7.6 mmol) and 4-chloroquinoline (1.49 g, 9.14 mmol) by an analogous procedure to that described in example 1. LC-MS (m/z) 316 (M+1).

Example 5

Preparation of 6-(8-methylquinolin-4-yloxy)-1-naphthoic acid

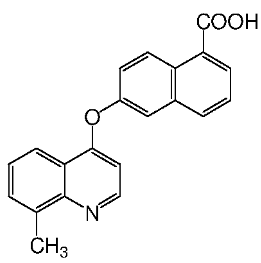

The title compound (1.25 g, 55% yield) was prepared as a brown solid from 6-hydroxy-1-naphthoic acid (1.43 g, 7.6 mmol) and 4-chloro-8-methylquinoline (1.62 g, 9.14 mmol) by an analogous procedure to that described in example 1. LC-MS (m/z) 330 (M+1).

Example 6

Preparation of 6-(7-chloroquinolin-4-yloxy)-1-naphthoic acid

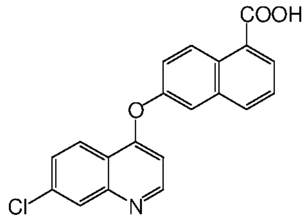

The title compound (1.57 g, 59% yield) was prepared as a brown solid from 6-hydroxy-1-naphthoic acid (1.43 g, 7.6 mmol) and 4,7-dichloroquinoline (1.81 g, 9.14 mmol) by an analogous procedure to that described in example 1. LC-MS (m/z) 350 (M+1).

Example 7

Preparation of 6-(8-(trifluoromethyl)quinolin-4-yloxy)-1-naphthoic acid

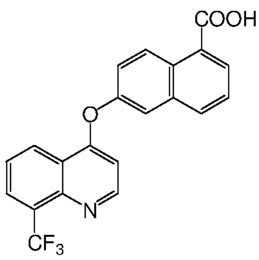

The title compound (1.43 g, 49% yield) was prepared as a brown solid from 6-hydroxy-1-naphthoic acid (1.43 g, 7.6 mmol) and 4-chloro-8-(trifluoromethyl)quinoline (2.12 g, 9.14 mmol) by an analogous procedure to that described in example 1. LC-MS (m/z) 384 (M+1).

Example 8

Preparation of 4-(aminomethyl)-N-(2-aminophenyl)benzamide

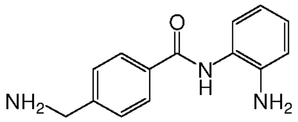

4-Cyanobenzoic acid (294 mg, 2 mmol) was dissolved in 8 ml of DMF, then 1-ethyl-3-(3-dimethyllaminopropyl)carbodiimide hydrochloride (768 mg, 4 mmol), 1-hydroxybenzotriazole (324 mg, 2.4 mmol), triethylamine (808 mg, 8 mmol) and o-phenylenediamine (432 mg, 4 mmol) were added. The mixture was stirred for 20 hours at room temperature. The mixture was diluted with 400 mL of brine. The solids were collected by vacuum filtration, washed with water and dried under vacuum to give N-(2-aminophenyl)-4-cyanobenzamide (364 mg, 77%) as a grey solid. LC-MS (m/z) 238 (M+1).
N-(2-aminophenyl)-4-cyanobenzamide (237 mg, 1 mmol) was dissolved in methanol (40 ml), then sulfuric acid (196 mg, 1 mmol) and 5% palladium on charcoal (0.20 g) were added. The mixture was stirred under an atmosphere of hydrogen until the reaction was finished. The mixture was filtered through celite, and the filtrate was neutralized with 1 N NaOH solution (2 ml). The resulting mixture was filtered, and the filtrate was concentrated under vacuum to give the title compound (232 mg, 96% yield) as a grey solid. LC-MS (m/z) 242 (M+1).

Example 9

Preparation of 4-(aminomethyl)-N-(2-amino-4-fluorophenyl)benzamide

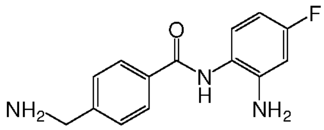

The title compound (186 mg, 72% yield) was prepared as a brown solid from 4-cyano-benzoic acid (294 mg, 2 mmol) and 4-fluoro-o-phenylenediamine (302 mg, 2.4 mmol) by an analogous procedure to that described in example 8. LC-MS (m/z) 260 (M+1).

Example 10

Preparation of 4-(aminomethyl)-N-(2-amino-4-methylphenyl)benzamide

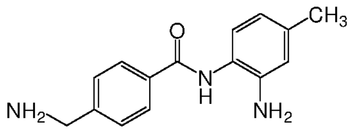

The title compound (173 mg, 68% yield) was prepared as a grey solid from 4-cyano-benzoic acid (294 mg, 2 mmol) and 4-methyl-o-phenylenediamine (293 mg, 2.4 mmol) by an analogous procedure to that described in example 8. LC-MS (m/z) 256 (M+1).

Example 11

Preparation of 4-(aminomethyl)-N-(2-amino-4-methoxyphenyl)benzamide

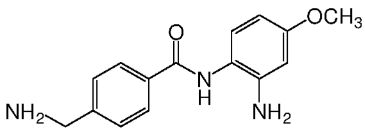

The title compound (192 mg, 71% yield) was prepared as a grey solid from 4-cyano-benzoic acid (294 mg, 2 mmol) and 4-methoxy-o-phenylenediamine (331 mg, 2.4 mmol) by an analogous procedure to that described in example 8. LC-MS (m/z) 272 (M+1).

Example 12

Preparation of 4-(aminomethyl)-N-(2-amino-4-trifluoromethylphenyl)benzamide

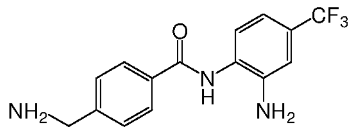

The title compound (195 mg, 63% yield) was prepared as a grey solid from 4-cyano-benzoic acid (294 mg, 2 mmol) and 4-trifluoromethyl-o-phenylenediamine (422 mg, 2.4 mmol) by an analogous procedure to that described in example 8. LC-MS (m/z) 310 (M+1).

Example 13

Preparation of 3-(aminomethyl)-N-(2-aminophenyl)benzamide

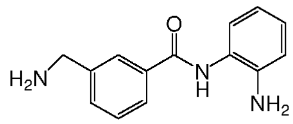

The title compound (140 mg, 58% yield) was prepared as a grey solid from 3-cyano-benzoic acid (294 mg, 2 mmol) and o-phenylenediamine (432 mg, 4 mmol) by an analogous procedure to that described in example 8. LC-MS (m/z) 242 (M+1).

Example 14

Preparation of 6-(aminomethyl)-N-(2-aminophenyl)nicotinamide

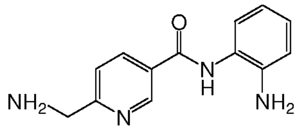

The title compound (157 mg, 65% yield) was prepared as a grey solid from 6-cyano-nicotinic acid (296 mg, 2 mmol) and o-phenylenediamine (864 mg, 8 mmol) by an analogous procedure to that described in example 8. LC-MS (m/z) 243(M+1).

Example 15

Preparation of 6-(aminomethyl)-N-(2-amino-4-fluorophenyl)nicotinamide

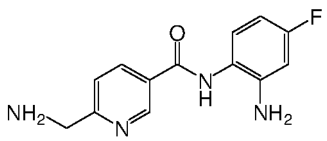

The title compound (135 mg, 52% yield) was prepared as a grey solid from 6-cyano-nicotinic acid (296 mg, 2 mmol) and 4-fluoro-o-phenylenediamine (302 mg, 2.4 mmol) by an analogous procedure to that described in example 8. LC-MS (m/z) 261 (M+1).

Example 16

Preparation of N-(2-aminophenyl)-6-(6,7-dimethoxyquinazolin-4-yloxy)-1-naphthamide

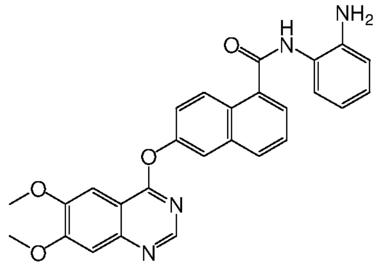

6-(6,7-Dimethoxyquinazolin-4-yloxy)-1-naphthoic acid (37.6 mg, 0.1 mmol) was dissolved in 4 ml of DMF, then 1-ethyl-3-(3-dimethyllaminopropyl)carbodi-imide hydrochloride (38.4 mg, 0.2 mmol), 1-hydroxybenzotriazole (16.2 mg, 0.12 mmol), triethylamine (40.4 mg, 0.4 mmol) and o-phenylenediamine (43.2 mg, 0.4 mmol) were added. The mixture was stirred for 20 hours at room temperature. The mixture was diluted with 200 mL of brine. The solids were collected by vacuum filtration, washed with water and dried under vacuum to give the title compound (39.1 mg, 84%) as a brown solid. $^1$H NMR (DMSO-$d_6$) $\delta$ 4.01 (s, 6H, 2 $\times$ $OCH_3$), 4.97 (s, 2H, benzene-$NH_2$), 6.65 (t, J= 7.2 Hz, 1 H, Ar-H), 6.82 (d, J= 7.0 Hz, 1H, Ar-H), 7.00 (t, J= 7.1 Hz, 1H, Ar-H), 7.38 (d, J= 7.1 Hz, 1H, Ar-H), 7.42 (s, 1H, Ar-H), 7.60 (dd, J= 2.4 and 9.2 Hz, 1 H, Ar-H), 7.64-7.68 (m, 2H, Ar-H), 7.87 (d, J= 6.7 Hz, 1 H, Ar-H), 7.97 (d, J= 2.3 Hz, 1 H, Ar-H), 8.09 (d, J= 8.2 Hz, 1 H, Ar-H), 8.38 (d, J= 9.2 Hz, 1 H, Ar-H), 8.54 (s, 1 H, Ar-H), 9.85 (s, 1 H, benzene-NH). LC-MS (m/z) 467 (M+1).

Example 17

Preparation of N-(2-amino-4-fluorophenyl)-6-(6,7-dimethoxyquinazolin-4-yloxy)-1-naphthamide

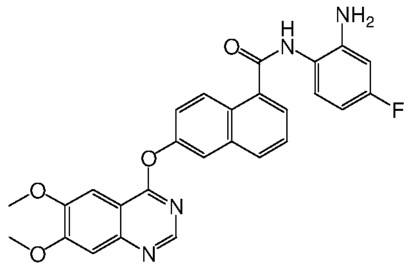

The title compound (43.1 mg, 89% yield) was prepared as a brown solid from 6-(6,7-dimethoxyquinazolin-4-yloxy)-1-naphthoic acid (37.6 mg, 0.1 mmol) and 4-fluoro-o-phenylenediamine (15.1 mg, 0.12 mmol) by an analogous procedure to that described in example 16. $^1$H NMR (DMSO-$d_6$) $\delta$ 4.01 (s, 6H, 2 $\times$ $OCH_3$), 5.28 (s, 2H, benzene-$NH_2$), 6.41 (td, J= 2.6 and 8.5 Hz, 1 H, Ar-H), 6.59 (dd, J= 2.6 and 11.2 Hz, 1 H, Ar-H), 7.35 (td, J= 1.8 and 7.5 Hz, 1 H, Ar-H), 7.41 (s, 1 H, Ar-H), 7.59 (dd, J= 2.2 and 8.4 Hz, 1 H, Ar-H), 7.63-7.67 (m, 2H, Ar-H), 7.89 (d, J= 6.9 Hz, 1 H, Ar-H), 7.96 (d, J= 1.9 Hz, 1 H, Ar-H), 8.08 (d, J= 8.2 Hz, 1 H, Ar-H), 8.38 (d, J= 9.2 Hz, 1 H, Ar-H), 8.54 (s, 1 H, Ar-H), 9.77 (s, 1 H, benzene-NH). LC-MS (m/z) 485 (M+1).

Example 18

Preparation of N-(2-amino-4-methylphenyl)-6-(6,7-dimethoxyquinazolin-4-yloxy)-1-naphthamide

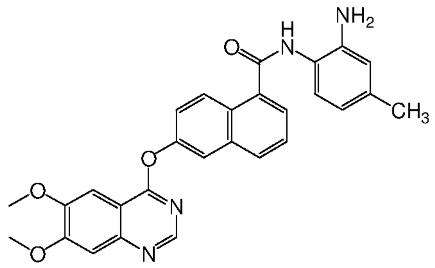

The title compound (39.4 mg, 82% yield) was prepared as a brown solid from 6-(6,7-dimethoxyquinazolin-4-yloxy)-1-naphthoic acid (37.6 mg, 0.1 mmol) and 4-methyl-o-phenylenediamine (14.6 mg, 0.12 mmol) by an analogous procedure to that described in example 16. $^1H$ NMR (DMSO-$d_6$) (isomer ratio 0.77/0.23) $\delta$ 2.21 (s, 1H, Ar-$CH_3$), 4.01 (s, 6H, 2 $\times$ $OCH_3$), 4.77 (s, 0.23x2H, benzene-$NH_2$), 4.89 (s, 0.77$\times$2H, benzene-$NH_2$), 6.46 (d, J= 7.6 Hz, 0.77$\times$1H, Ar-H), 6.64 (s, 0.77$\times$1H, Ar-H), 6.73 (d, J= 7.9 Hz, 0.23$\times$1H, Ar-H), 6.81 (s, 0.23$\times$1H, Ar-H), 7.24 (d, J= 8.1 Hz, 1H, Ar-H), 7.41 (s, 1H, Ar-H), 7.58-7.66 (m, 3H, Ar-H), 7.85 (d, J= 6.7 Hz, 1H, Ar-H), 7.97 (s, 1H, Ar-H), 8.08 (d, J= 7.9 Hz, 1H, Ar-H), 8.38 (d, J= 9.0 Hz, 1H, Ar-H), 8.54 (s, 1H, Ar-H), 9.77 (s, 1H, benzene-NH). LC-MS (m/z) 481 (M+1).

Example 19

Preparation of N-(2-amino-4-methoxyphenyl)-6-(6,7-dimethoxyquinazolin-4-yloxy)-1-naphthamide

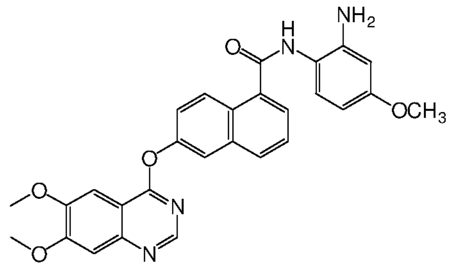

The title compound (43.2 mg, 87% yield) was prepared as a brown solid from 6-(6,7-dimethoxyquinazolin-4-yloxy)-1-naphthoic acid (37.6 mg, 0.1 mmol) and 4-methoxy-o-phenylenediamine (16.5 mg, 0.12 mmol) by an analogous procedure to that described in example 16. $^1H$ NMR (DMSO-$d_6$) $\delta$ 3.70 (s, 3H, -$OCH_3$), 4.01 (s, 6H, 2 $\times$ $OCH_3$), 5.00 (s, 2H, benzene-$NH_2$), 6.23 (dd, J= 2.6 and 8.6 Hz, 1 H, Ar-H), 6.40 (d, J= 2.6 Hz, 1 H, Ar-H), 7.22 (d, J= 8.6 Hz, 1 H, Ar-H), 7.41 (s, 1 H, Ar-H), 7.59 (dd, J= 2.2 and 9.1 Hz, 1H, Ar-H), 7.62-7.66 (m, 2H, Ar-H), 7.86 (d, J= 6.9 Hz, 1H, Ar-H), 7.96 (d, J= 2.0 Hz, 1 H, Ar-H), 8.07 (d, J= 8.2 Hz, 1 H, Ar-H), 8.38 (d, J= 9.2 Hz, 1 H, Ar-H), 8.54 (s, 1 H, Ar-H), 9.70 (s, 1 H, benzene-NH). LC-MS (m/z) 497 (M+1).

Example 20

Preparation of N-(2-amino-4-chlorophenyl)-6-(6,7-dimethoxyquinazolin-4-yloxy)-1-naphthamide

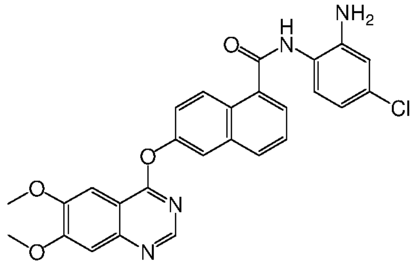

The title compound (42.9 mg, 83% yield) was prepared as a brown solid from 6-(6,7-dimethoxyquinazolin-4-yloxy)-1-naphthoic acid (37.6 mg, 0.1 mmol) and 4-chloro-o-phenylenediamine (17.1 mg, 0.12 mmol) by an analogous procedure to that described in example 16. $^1$H NMR (DMSO-$d_6$) δ 4.01 (s, 6H, 2 × $OCH_3$), 5.31 (s, 2H, benzene-$NH_2$), 6.65 (d, J= 8.3 Hz, 1H, Ar-H), 6.86 (d, J= 1.9 Hz, 1H, Ar-H), 7.41 (s, 1H, Ar-H), 7.58-7.67 (m, 4H, Ar-H), 7.89 (d, J= 6.8 Hz, 1H, Ar-H), 8.01 (s, 1H, Ar-H), 8.09 (d, J= 8.1 Hz, 1H, Ar-H), 8.37 (d, J= 9.2 Hz, 1H, Ar-H), 8.55 (s, 1H, Ar-H), 9.84 (s, 1H, benzene-NH). LC-MS (m/z) 501 (M+1).

Example 21

Preparation of N-(2-amino-4-bromophenyl)-6-(6,7-dimethoxyquinazolin-4-yloxy)-1-naphthamide

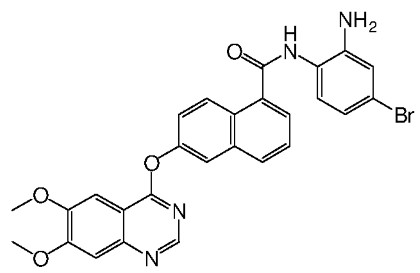

The title compound (42.0 mg, 77% yield) was prepared as a brown solid from 6-(6,7-dimethoxyquinazolin-4-yloxy)-1-naphthoic acid (37.6 mg, 0.1 mmol) and 4-bromo-o-phenylenediamine (22.4 mg, 0.12 mmol) by an analogous procedure to that described in example 16. $^1$H NMR (DMSO-$d_6$) δ 4.01 (s, 6H, 2 × $OCH_3$), 5.31 (s, 2H, benzene-$NH_2$), 6.77 (d, J= 8.3 Hz, 1H, Ar-H), 7.01 (s, 1H, Ar-H), 7.41 (s, 1H, Ar-H), 7.58-7.65 (m, 5H, Ar-H), 7.89 (d, J= 7.0 Hz, 1H, Ar-H), 8.00 (s, 1 H, Ar-H), 8.14 (d, J= 10.2 Hz, 1 H, Ar-H), 8.37 (d, J= 9.1 Hz, 1 H, Ar-H), 8.54 (s, 1 H, Ar-H), 9.84 (s, 1 H, benzene-NH). LC-MS (m/z) 545 (M+1).

Example 22

Preparation of N-(2-amino-4-trifluoromethylphenyl)-6-(6,7-dimethoxyquinazolin-4-yloxy)-1-naphthamide

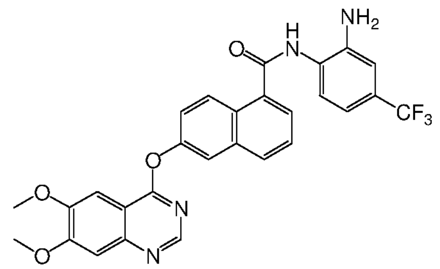

The title compound (42.3 mg, 79% yield) was prepared as a brown solid from 6-(6,7-dimethoxyquinazolin-4-yloxy)-1-naphthoic acid (37.6 mg, 0.1 mmol) and 4-trifluoromethyl-o-phenylenediamine (21.1 mg, 0.12 mmol) by an analogous procedure to that described in example 16. $^1$H NMR (DMSO-$d_6$) δ 4.01 (s, 6H, 2 × $OCH_3$), 5.72 (s, 2H, benzene-$NH_2$), 6.92 (d, J= 8.5 Hz, 1H, Ar-H), 7.42 (s, 1H, Ar-H), 7.59-7.65 (m, 3H, Ar-H), 7.90-7.96 (m, 2H, Ar-H), 7.98 (s, 1H, Ar-H), 8.10 (d, J= 8.3 Hz, 1H, Ar-H), 8.17 (d, J= 7.3 Hz, 1 H, Ar-H), 8.39 (d, J= 9.2 Hz, 1 H, Ar-H), 8.54 (s, 1 H, Ar-H), 9.90 (s, 1 H, benzene-NH). LC-MS (m/z) 535 (M+1).

Example 23

Preparation of N-(4-((2-aminophenyl)carbamoyl)benzyl)-6-(6,7-dimethoxyquinazolin-4-yloxy)-1-naphthamide

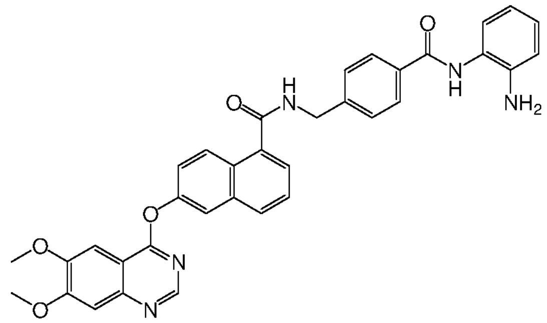

The title compound (43.1 mg, 72% yield) was prepared as a brown solid from 6-(6,7-dimethoxyquinazolin-4-yloxy)-1-naphthoic acid (37.6 mg, 0.1 mmol) and 4-(aminomethyl)-N-(2-aminophenyl)benzamide (28.9 mg, 0.12 mmol) by an analogous procedure to that described in example 16. LC-MS (m/z) 600 (M+1).

Example 24

Preparation of N-(4-((2-amino-4-fluorophenyl)carbamoyl)benzyl)-6-(6,7-dimethoxyquinazolin-4-yloxy)-1-naphthamide

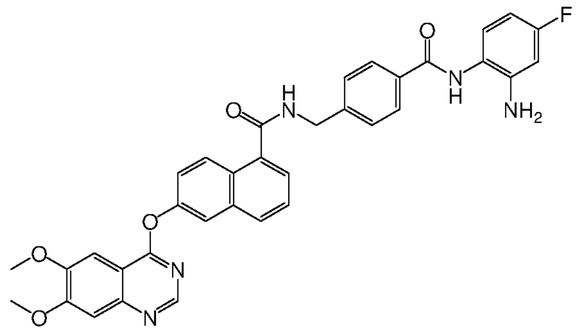

The title compound (46.3 mg, 75% yield) was prepared as a brown solid from 6-(6,7-dimethoxyquinazolin-4-yloxy)-1-naphthoic acid (37.6 mg, 0.1 mmol) and 4-(aminomethyl)-N-(2-amino-4-fluorophenyl)benzamide (31.1 mg, 0.12 mmol) by an analogous procedure to that described in example 16. LC-MS (m/z) 618 (M+1).

Example 25

Preparation of N-(2-aminophenyl)-6-((2-(6,7-dimethoxyquinazolin-4-yloxy)-1-naphthamido)methyl)nicotinamide

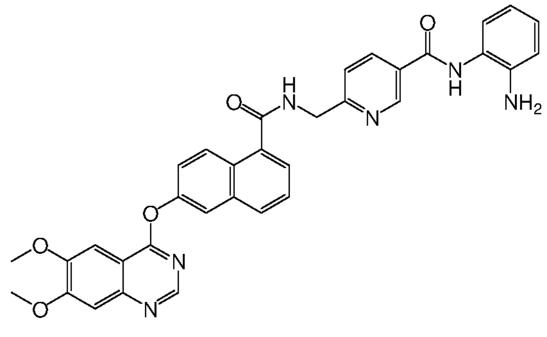

The title compound (41.4 mg, 69% yield) was prepared as a brown solid from 6-(6,7-dimethoxyquinazolin-4-yloxy)-1-naphthoic acid (37.6 mg, 0.1 mmol) and 6-(aminomethyl)-N-(2-aminophenyl)nicotinamide (29.0 mg, 0.12 mmol)

by an analogous procedure to that described in example 16. LC-MS (m/z) 601 (M+1).

Example 26

Preparation of N-(2-amino-4-fluorophenyl)-6-((2-(6,7-dimethoxyquinazolin-4-yloxy)-1-naphthamido)methyl)nicotinamide

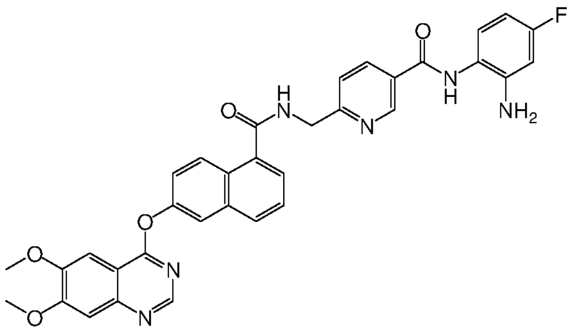

The title compound (43.3 mg, 77% yield) was prepared as a brown solid from 6-(6,7-dimethoxyquinazolin-4-yloxy)-1-naphthoic acid (37.6 mg, 0.1 mmol) and 6-(aminomethyl)-N-(2-amino-4-fluorophenyl)nicotinamide (31.2 mg, 0.12 mmol) by an analogous procedure to that described in example 16. LC-MS (m/z) 619 (M+1).

Example 27

Preparation of N-(3-((2-aminophenyl)carbamoyl)benzyl)-6-(6,7-dimethoxyquinazolin-4-yloxy)-1-naphthamide

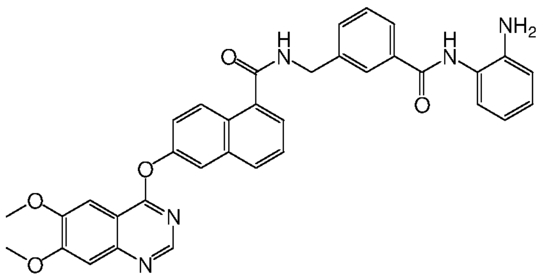

The title compound (48.5 mg, 81% yield) was prepared as a brown solid from 6-(6,7-dimethoxyquinazolin-4-yloxy)-1-naphthoic acid (37.6 mg, 0.1 mmol) and 3-(aminomethyl)-N-(2-aminophenyl)benzamide (28.9 mg, 0.12 mmol) by an analogous procedure to that described in example 16. LC-MS (m/z) 600 (M+1).

Example 28

Preparation of N-(4-((2-amino-4-methylphenyl)carbamoyl)benzyl)-6-(6,7-dimethoxyquinazolin-4-yloxy)-1-naphthamide

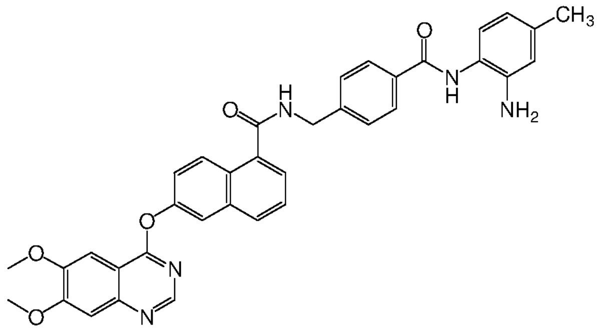

The title compound (52.7 mg, 86% yield) was prepared as a brown solid from 6-(6,7-dimethoxyquinazolin-4-yloxy)-1-naphthoic acid (37.6 mg, 0.1 mmol) and 4-(aminomethyl)-N-(2-amino-4-methylphenyl)benzamide (30.6 mg, 0.12 mmol) by an analogous procedure to that described in example 16. LC-MS (m/z) 614 (M+1).

Example 29

Preparation of N-(4-((2-amino-4-methoxyphenyl)carbamoyl)benzyl)-6-(6,7-dimethoxyquinazolin-4-yloxy)-1-naphthamide

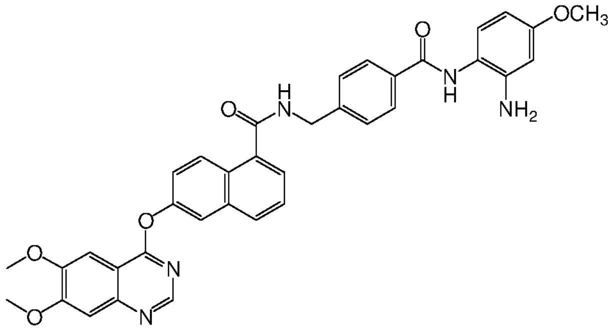

The title compound (51.6 mg, 82% yield) was prepared as a brown solid from 6-(6,7-dimethoxyquinazolin-4-yloxy)-1-naphthoic acid (37.6 mg, 0.1 mmol) and 4-(aminomethyl)-N-(2-amino-4-methoxyphenyl)benzamide (32.5 mg, 0.12 mmol) by an analogous procedure to that described in example 16. LC-MS (m/z) 630 (M+1).

Example 30

Preparation of N-(4-((2-amino-4-trifluoromethylphenyl)carbamoyl)benzyl)-6-(6,7-dimethoxyquinazolin-4-yloxy)-1-naphthamide

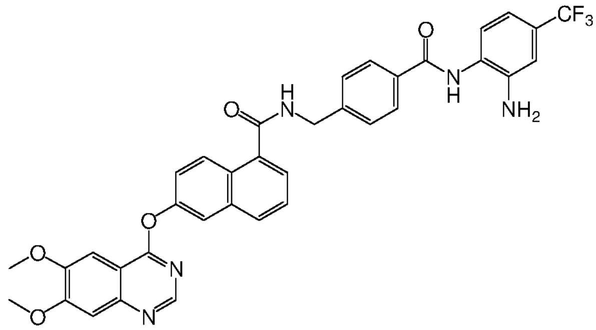

The title compound (46.7 mg, 70% yield) was prepared as a brown solid from 6-(6,7-dimethoxyquinazolin-4-yloxy)-1-naphthoic acid (37.6 mg, 0.1 mmol) and 4-(aminomethyl)-N-(2-amino-4-trifluoromethylphenyl)benzamide (37.1 mg, 0.12 mmol) by an analogous procedure to that described in example 16. LC-MS (m/z) 668 (M+1).

Example 31

Preparation of N-(2-aminophenyl)-6-(7-methoxyquinolin-4-yloxy)-1-naphthamide

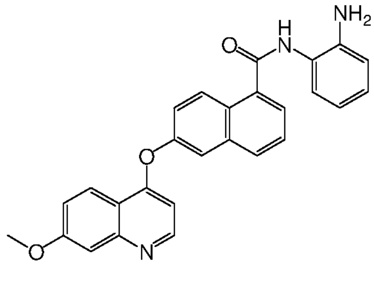

The title compound (39.6 mg, 91% yield) was prepared as a brown solid from 6-(7-methoxyquinolin-4-yloxy)-1-naphthoic acid (34.5 mg, 0.1 mmol) and o-phenylenediamine (43.2 mg, 0.4 mmol) by an analogous procedure to that described in example 16. $^1$H NMR (DMSO-$d_6$) $\delta$ 3.95 (s, 3H, -$OCH_3$), 4.97 (s, 2H, benzene-$NH_2$), 6.60 (d, J= 5.2 Hz, 1 H, Ar-H), 6.64 (t, J= 7.6 Hz, 1 H, Ar-H), 6.82 (d, J= 7.8 Hz, 1 H, Ar-H), 6.99 (t, J= 7.4 Hz, 1 H, Ar-H), 7.31 (dd, J= 2.5 and 9.1 Hz, 1 H, Ar-H), 7.38 (d, J= 7.6 Hz, 1 H, Ar-H), 7.45 (d, J= 2.4 Hz, 1 H, Ar-H), 7.57 (dd, J= 2.4 and 9.2 Hz, 1 H, Ar-H), 7.65 (t, J= 7.8 Hz, 1 H, Ar-H), 7.87-7.88 (m, 2H, Ar-H), 8.07 (d, J= 8.2 Hz, 1 H, Ar-H), 8.25 (d, J= 9.2 Hz, 1 H, Ar-H), 8.43 (d, J= 9.2 Hz, 1H, Ar-H), 8.65 (d, J= 5.2 Hz, 1H, Ar-H), 9.84 (s, 1H, benzene-NH). 436 (M+1).

Example 32

Preparation of N-(2-amino-4-fluorophenyl)-6-(7-methoxyquinolin-4-yloxy)-1-naphthamide

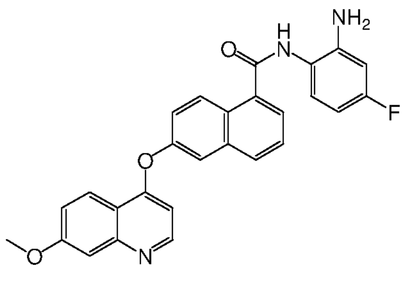

The title compound (33.1 mg, 73% yield) was prepared as a brown solid from 6-(7-methoxyquinolin-4-yloxy)-1-naphthoic acid (34.5 mg, 0.1 mmol) and 4-fluoro-o-phenylenediamine (15.1 mg, 0.12 mmol) by an analogous procedure to that described in example 16. $^1$H NMR (DMSO-$d_6$) $\delta$ 3.95 (s, 3H, -$OCH_3$), 5.27 (s, 2H, benzene-$NH_2$), 6.41 (td, J= 2.5 and 8.4 Hz, 1H, Ar-H), 6.57-6.61 (m, 2H, Ar-H), 7.30-7.36 (m, 2H, Ar-H), 7.45 (d, J= 2.2 Hz, 1 H, Ar-H), 7.56 (dd, J= 2.2 and 9.2 Hz, 1 H, Ar-H), 7.65 (t, J= 7.6 Hz, 1H, Ar-H), 7.87-7.91 (m, 2H, Ar-H), 8.07 (d, J= 8.3 Hz, 1H, Ar-H), 8.24 (d, J= 9.1 Hz, 1 H, Ar-H), 8.43 (d, J= 9.2 Hz, 1 H, Ar-H), 8.65 (d, J= 5.1 Hz, 1 H, Ar-H), 9.75 (s, 1 H, benzene-NH). LC-MS (m/z) 454 (M+1).

Example 33

Preparation of N-(4-((2-aminophenyl)carbamoyl)benzyl)-6-(7-methoxyquinolin-4-yloxy)-1-naphthamide

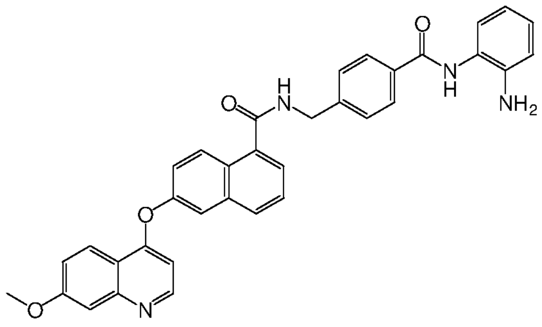

The title compound (48.3 mg, 85% yield) was prepared as a brown solid from 6-(7-methoxyquinolin-4-yloxy)-1-naphthoic acid (34.5 mg, 0.1 mmol) and 4-(aminomethyl)-N-(2-aminophenyl)benzamide (28.9 mg, 0.12 mmol) by an analogous procedure to that described in example 16. $^1$H NMR (DMSO-$d_6$) $\delta$ 3.95 (s, 3H, -$OCH_3$), 4.64 (d, J= 5.6 Hz, 2H, -$CH_2$), 4.87 (s, 2H, benzene-$NH_2$), 6.58-6.62 (m, 2H, Ar-H), 6.78 (dd, J= 1.2 and 7.8 Hz, 1H, Ar-H), 6.97 (td, J= 1.4 and 8.1 Hz, 1H, Ar-H), 7.18 (d, J= 7.0 Hz, 1H, Ar-H), 7.31 (dd, J= 2.5 and 9.2 Hz, 1H, Ar-H), 7.44 (d, J= 2.4 Hz, 1H, Ar-H), 7.53-7.56 (m, 3H, Ar-H), 7.62 (t, J= 8.0 Hz, 1 H, Ar-H), 7.72 (d, J= 6.1 Hz, 1 H, Ar-H), 7.86 (d, J= 2.5 Hz, 1 H, Ar-H), 7.98-8.06 (m, 3H, Ar-H), 8.24 (d, J= 9.1 Hz, 1 H, Ar-H), 8.39 (d, J= 9.2 Hz, 1 H, Ar-H), 8.64 (d, J= 5.2 Hz, 1 H, Ar-H), 9.21 (t, J= 6.0 Hz, 1 H, -CONH), 9.61 (s, 1H, benzene-NH). LC-MS (m/z) 569 (M+1).

Example 34

Preparation of N-(2-aminophenyl)-6-((2-(7-methoxyquinolin-4-yloxy)-1-naphthamido)methyl)nicotinamide

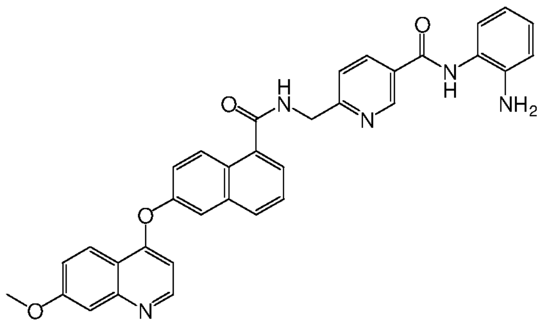

The title compound (46.6 mg, 82% yield) was prepared as a brown solid from 6-(7-methoxyquinolin-4-yloxy)-1-naphthoic acid (34.5 mg, 0.1 mmol) and 6-(aminomethyl)-N-(2-aminophenyl)nicotinamide (29.0 mg, 0.12 mmol) by an analogous procedure to that described in example 16. $^1$H NMR (DMSO-$d_6$) $\delta$ 3.95 (s, 3H, -$OCH_3$), 4.74 (s, 2H, -$CH_2$), 4.95 (s, 2H, benzene-$NH_2$), 6.60 (m, 2H, Ar-H), 6.79 (s, 1 H, Ar-H), 6.98 (s, 1H, Ar-H), 7.17 (s, 1H, Ar-H), 7.31 (d, J= 8.6 Hz, 1H, Ar-H), 7.44 (s, 1H, Ar-H), 7.58-7.63 (m, 3H, Ar-H), 7.77 (s, 1H, Ar-H), 7.87 (s, 1H, Ar-H), 8.05 (d, J= 5.6 Hz, 1H, Ar-H), 8.24 (d, J= 8.3 Hz, 1 H, Ar-H), 8.33 (s, 1 H, Ar-H), 8.47 (d, J= 7.5 Hz, 1 H, Ar-H), 9.13 (s, 1 H, Ar-H), 9.25 (s, 1 H, -CONH), 9.77 (s, 1 H, benzene-NH). LC-MS (m/z) 570 (M+1).

Example 35

Preparation of N-(2-aminophenyl)-6-(6,7-dimethoxyquinolin-4-yloxy)-1-naphthamide

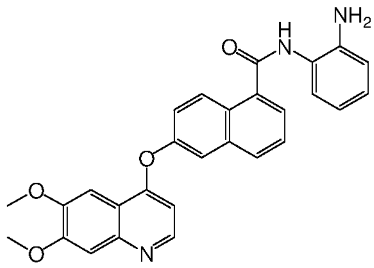

The title compound (40.0 mg, 86% yield) was prepared as a brown solid from 6-(6,7-dimethoxyquinolin-4-yloxy)-1-naphthoic acid (37.5 mg, 0.1 mmol) and o-phenylenediamine (43.2 mg, 0.4 mmol) by an analogous procedure to that described in example 16. $^1$H NMR (DMSO-$d_6$) $\delta$ 3.93 (s, 3H, -$OCH_3$), 3.95 (s, 3H, -$OCH_3$), 4.99 (s, 2H, benzene-$NH_2$), 6.56 (d, J= 5.2 Hz, 1 H, Ar-H), 6.63 (t, J= 7.6 Hz, 1 H, Ar-H), 6.81 (d, J= 7.6 Hz, 1H, Ar-H), 6.98 (t, J= 7.2 Hz, 1H, Ar-H), 7.36 (d, J= 7.6 Hz, 1H, Ar-H), 7.43 (s, 1H, Ar-H), 7.56-7.58 (m, 2H, Ar-H), 7.65 (t, J= 7.6 Hz, 1 H, Ar-H), 7.87-7.90 (m, 2H, Ar-H), 8.08 (d, J= 8.0 Hz, 1 H, Ar-H), 8.43 (d, J= 9.2 Hz, 1 H, Ar-H), 8.49 (d, J= 5.2 Hz, 1 H, Ar-H), 9.87 (s, 1 H, benzene-NH). LC-MS (m/z) 466 (M+1).

Example 36

Preparation of N-(2-amino-4-fluorophenyl)-6-(6,7-dimethoxyquinolin-4-yloxy)-1-naphthamide

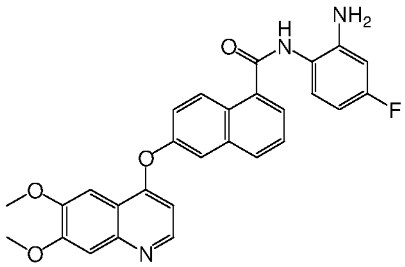

The title compound (39.1 mg, 81% yield) was prepared as a brown solid from 6-(6,7-dimethoxyquinolin-4-yloxy)-1-naphthoic acid (37.5 mg, 0.1 mmol) and 4-fluoro-o-phenylenediamine (15.1 mg, 0.12 mmol) by an analogous procedure to that described in example 16. $^1$H NMR (DMSO-$d_6$) $\delta$ 3.93 (s, 3H, -$OCH_3$), 3.95 (s, 3H, -$OCH_3$), 5.31 (s, 2H, benzene-$NH_2$), 6.40 (s, 1 H, Ar-H), 6.55-6.59 (m, 2H, Ar-H), 7.30 (d, J= 7.6 Hz, 1H, Ar-H), 7.42 (s, 1H, Ar-H), 7.54-7.57 (m, 2H, Ar-H), 7.64 (t, J= 8.0 Hz, 1H, Ar-H), 7.89-7.91 (m, 2H, Ar-H), 8.07 (d, J= 8.0 Hz, 1 H, Ar-H), 8.42 (d, J= 9.2 Hz, 1 H, Ar-H), 8.49 (d, J= 5.2 Hz, 1 H, Ar-H), 9.79 (s, 1 H, benzene-NH). LC-MS (m/z) 484 (M+1).

Example 37

Preparation of N-(4-((2-aminophenyl)carbamoyl)benzyl)-6-(6,7-dimethoxyquinolin-4-yloxy)-1-naphthamide

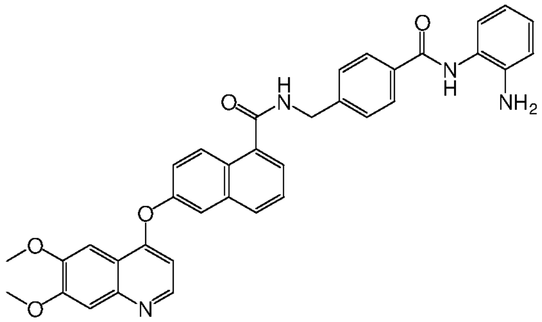

The title compound (49.0 mg, 82% yield) was prepared as a brown solid from 6-(6,7-dimethoxyquinolin-4-yloxy)-1-naphthoic acid (37.5 mg, 0.1 mmol) and 4-(aminomethyl)-N-(2-aminophenyl)benzamide (28.9 mg, 0.12 mmol) by an analogous procedure to that described in example 16. $^1$H NMR (DMSO-$d_6$) $\delta$ 3.93 (s, 3H, -$OCH_3$), 3.95 (s, 3H, -$OCH_3$), 4.63 (d, J= 5.6 Hz, 2H, -$CH_2$), 4.90 (s, 2H, benzene-$NH_2$), 6.56-6.59 (m, 2H, Ar-H), 6.78 (d, J= 7.6 Hz, 1 H, Ar-H), 6.96 (t, J= 8.1 Hz, 1 H, Ar-H), 7.17 (d, J= 7.6 Hz, 1 H, Ar-H), 7.42 (s, 1 H, Ar-H), 7.53-7.55 (m, 4H, Ar-H), 7.62 (t, J= 8.0 Hz, 1 H, Ar-H), 7.71 (d, J= 6.8 Hz, 1 H, Ar-H), 7.87 (s, 1 H, Ar-H), 7.98-8.06 (m, 3H, Ar-H), 8.39 (d, J= 9.2 Hz, 1 H, Ar-H), 8.49 (d, J= 5.2 Hz, 1 H, Ar-H), 9.26 (t, J= 6.0 Hz, 1 H, -CONH), 9.66 (s, 1 H, benzene-NH). LC-MS (m/z) 599 (M+1).

Example 38

Preparation of N-(2-aminophenyl)-6-((2-(6,7-dimethoxyquinolin-4-yloxy)-1-naphthamido)methyl)nicotinamide

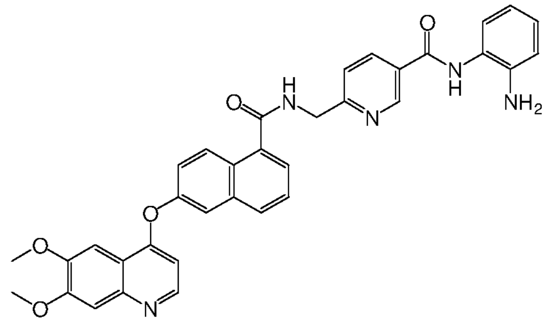

The title compound (47.9 mg, 80% yield) was prepared as a brown solid from 6-(6,7-dimethoxyquinolin-4-yloxy)-1-naphthoic acid (37.5 mg, 0.1 mmol) and 6-(aminomethyl)-N-(2-aminophenyl)nicotinamide (29.0 mg, 0.12 mmol) by an analogous procedure to that described in example 16. $^1$H NMR (DMSO-$d_6$) $\delta$ 3.93 (s, 3H, -$OCH_3$), 3.95 (s, 3H, -$OCH_3$), 4.73 (d, J= 5.6 Hz, 2H, -$CH_2$), 4.97 (s, 2H, benzene-$NH_2$), 6.57 (m, 2H, Ar-H), 6.77 (d, J= 6.4 Hz, 1H, Ar-H), 6.98 (t, J= 8.1 Hz, 1H, Ar-H), 7.16 (d, J= 5.6 Hz, 1 H, Ar-H), 7.42 (s, 1 H, Ar-H), 7.55-7.63 (m, 4H, Ar-H), 7.62 (t, J= 8.0 Hz, 1 H, Ar-H), 7.76 (d, J= 6.8 Hz, 1H, Ar-H), 7.88 (s, 1H, Ar-H), 8.06 (s, 1H, Ar-H), 8.33 (s, 1H, Ar-H), 8.45-8.48 (m, 2H, Ar-H), 9.12 (s, 1H, Ar-H), 9.30 (t, J= 6.0 Hz, 1H, -CONH), 9.80 (s, 1H, benzene-NH). LC-MS (m/z) 600 (M+1).

Example 39

Preparation of N-(2-aminophenyl)-6-(quinolin-4-yloxy)-1-naphthamide

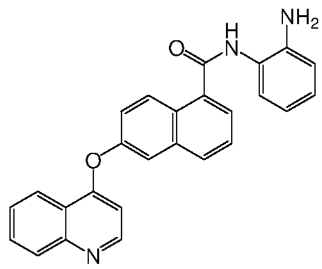

The title compound (35.6 mg, 88% yield) was prepared as a brown solid from 6-(quinolin-4-yloxy)-1-naphthoic acid (31.5 mg, 0.1 mmol) and o-phenylenediamine (43.2 mg, 0.4 mmol) by an analogous procedure to that described in example 16. $^1$H NMR (DMSO-$d_6$) $\delta$ 4.97 (s, 2H, benzene-$NH_2$), 6.65 (t, J= 7.3 Hz, 1 H, Ar-H), 6.75 (d, J= 5.1 Hz, 1 H, Ar-H), 6.82 (d, J= 7.8 Hz, 1 H, Ar-H), 7.00 (t, J= 7.1 Hz, 1 H, Ar-H), 7.38 (d, J= 7.5 Hz, 1H, Ar-H), 7.59 (dd, J= 2.3 and 9.2 Hz, 1H, Ar-H), 7.64-7.71 (m, 2H, Ar-H), 7.83-7.92 (m, 3H, Ar-H), 8.08 (d, J= 8.4 Hz, 2H, Ar-H), 8.37 (d, J= 7.9 Hz, 1 H, Ar-H), 8.45 (d, J= 9.2 Hz, 1H, Ar-H), 8.73 (d, J= 5.1 Hz, 1H, Ar-H), 9.85 (s, 1H, benzene-NH). LC-MS (m/z) 406 (M+1).

Example 40

Preparation of N-(2-aminophenyl)-6-(8-methylquinolin-4-yloxy)-1-naphthamide

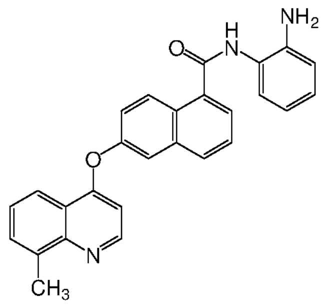

The title compound (37.7 mg, 90% yield) was prepared as a brown solid from 6-(8-methylquinolin-4-yloxy)-1-naphthoic acid (32.9 mg, 0.1 mmol) and o-phenylenediamine (43.2 mg, 0.4 mmol) by an analogous procedure to that described in example 16. $^1$H NMR (DMSO-$d_6$) $\delta$ 2.76 (s, 3H, Ar-$CH_3$), 4.97 (s, 2H, benzene-$NH_2$), 6.64 (t, J= 7.1 Hz, 1 H, Ar-H), 6.78 (d, J= 5.0 Hz, 1 H, Ar-H), 6.82 (d, J= 7.8 Hz, 1 H, Ar-H), 6.99 (t, J= 7.3 Hz, 1 H, Ar-H), 7.38 (d, J= 7.5 Hz, 1 H, Ar-H), 7.55-7.58 (m, 2H, Ar-H), 7.65 (t, J= 7.6 Hz, 1 H, Ar-H), 7.71 (d, J= 7.0 Hz, 1 H, Ar-H), 7.87-7.89 (m, 2H, Ar-H), 8.07 (d, J= 8.2 Hz, 1 H, Ar-H), 8.20 (d, J= 7.9 Hz, 1 H, Ar-H), 8.44 (d, J= 9.2 Hz, 1 H, Ar-H), 8.76 (d, J= 5.0 Hz, 1 H, Ar-H), 9.84 (s, 1 H, benzene-NH). LC-MS (m/z) 420 (M+1).

Example 41

Preparation of N-(2-aminophenyl)-6-(7-chloroquinolin-4-yloxy)-1-naphthamide

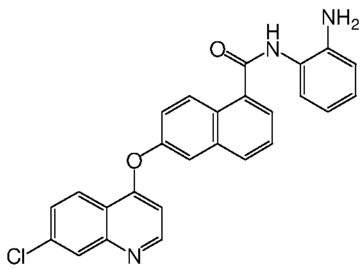

The title compound (33.2 mg, 83% yield) was prepared as a brown solid from 6-(7-chloroquinolin-4-yloxy)-1-naphthoic acid (35.0 mg, 0.1 mmol) and o-phenylenediamine (43.2 mg, 0.4 mmol) by an analogous procedure to that described in example 16. $^1$H NMR (DMSO-$d_6$) $\delta$ 4.97 (s, 2H, benzene-$NH_2$), 6.65 (t, J= 7.4 Hz, Ar-H), 6.77 (d, J= 5.5 Hz, 1 H, Ar-H), 6.82 (d, J= 7.2 Hz, 1 H, Ar-H), 7.00 (t, J= 7.0 Hz, 1 H, Ar-H), 7.38 (d, J= 7.2 Hz, 1H, Ar-H), 7.60 (dd, J= 2.6 and 9.2 Hz, 1H, Ar-H), 7.67-7.74 (m, 2H, Ar-H), 7.89 (d, J= 7.4 Hz, 1 H, Ar-H), 7.94 (d, J= 2.4 Hz, 1 H, Ar-H), 8.09 (d, J= 8.2 Hz, 1 H, Ar-H), 8.13 (d, J= 2.1 Hz, 1H, Ar-H), 8.41 (d, J= 9.0 Hz, 1 H, Ar-H), 8.46 (d, J= 9.6 Hz, 1H, Ar-H), 8.76 (d, J= 5.2 Hz, 1 H, Ar-H), 9.85 (s, 1 H, benzene-NH). LC-MS (m/z) 440 (M+1).

Example 42

Preparation of N-(2-aminophenyl)-6-(8-trifluoromethylquinolin-4-yloxy)-1-naphthamide

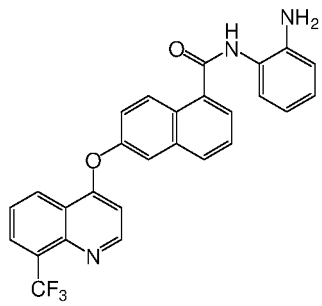

The title compound (38.3 mg, 81% yield) was prepared as a brown solid from 6-(8-trifluoromethylquinolin-4-yloxy)-1-naphthoic acid (39.8 mg, 0.1 mmol) and o-phenylenediamine (43.2 mg, 0.4 mmol) by an analogous procedure to that described in example 16. $^1$H NMR (DMSO-$d_6$) $\delta$ 4.98 (s, 2H, benzene-$NH_2$), 6.65 (t, J= 7.3 Hz, 1H, Ar-H), 6.83 (d, J= 7.6 Hz, 1H, Ar-H), 6.89 (d, J= 5.2 Hz, 1H, Ar-H), 7.00 (t, J= 7.2 Hz, 1H, Ar-H), 7.38 (d, J= 7.5 Hz, 1 H, Ar-H), 7.62 (dd, J= 2.4 and 9.2 Hz, 1 H, Ar-H), 7.68 (t, J= 7.7 Hz, 1 H, Ar-H), 7.83 (t, J= 7.9 Hz, 1 H, Ar-H), 7.90 (d, J= 7.0 Hz, 1 H, Ar-H), 7.97 (d, J= 2.3 Hz, 1 H, Ar-H), 8.10 (d, J= 8.3 Hz, 1 H, Ar-H), 8.29 (d, J= 7.1 Hz, 1 H, Ar-H), 8.47 (d, J= 9.2 Hz, 1H, Ar-H), 8.70 (d, J= 7.8 Hz, 1H, Ar-H), 8.87 (d, J= 5.2 Hz, 1H, Ar-H), 9.86 (s, 1H, benzene-NH). LC-MS (m/z) 474 (M+1).

Example 43

Preparation of N-(4-((2-aminophenyl)carbamoyl)benzyl)-6-(7-chloroquinolin-4-yloxy)-1-naphthamide

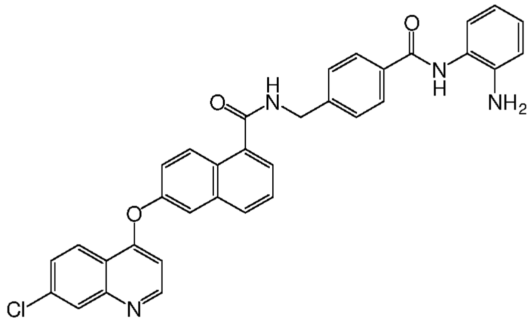

The title compound (42.4 mg, 74% yield) was prepared as a brown solid from 6-(7-chloroquinolin-4-yloxy)-1-naphthoic acid (35.0 mg, 0.1 mmol) and 4-(aminomethyl)-N-(2-aminophenyl)benzamide (28.9 mg, 0.12 mmol) by an analogous procedure to that described in example 16. $^1$H NMR (DMSO-$d_6$) $\delta$ 4.64 (d, J= 5.8 Hz, 2H, -$CH_2$), 4.87 (s, 2H, benzene-$NH_2$), 6.60 (t, J= 7.0 Hz, 1H, Ar-H), 6.75-6.79 (m, 2H, Ar-H), 6.97 (t, J= 7.5 Hz, 1 H, Ar-H), 7.18 (d, J= 7.7 Hz, 1 H, Ar-H), 7.53-7.59 (m, 3H, Ar-H), 7.66 (t, J= 8.0 Hz, 1H, Ar-H), 7.70-7.74 (m, 2H, Ar-H), 7.92 (d, J= 2.0 Hz, 1H, Ar-H), 7.99 (d, J= 7.9 Hz, 2H, Ar-H), 8.06 (d, J= 8.2 Hz, 1H, Ar-H), 8.13 (s, 1H, Ar-H), 8.39-8.42 (m, 2H, Ar-H), 8.75 (d, J= 5.1 Hz, 1H, Ar-H), 9.22 (t, J= 5.6 Hz, 1H, -CONH), 9.62 (s, 1H, benzene-NH). LC-MS (m/z) 573 (M+1).

Example 44

Preparation of N-(4-(((2-aminophenyl)carbamoyl)benzyl)-6-(8-trifluoromethylquinolin-4-yloxy)-1-naphthamide

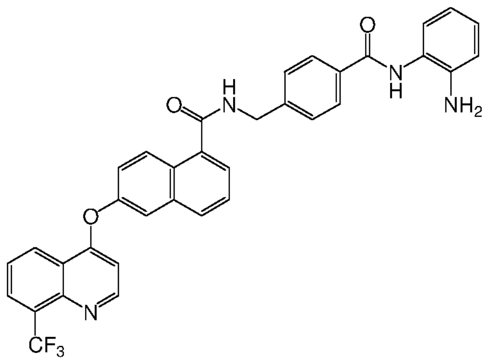

The title compound (47.3 mg, 78% yield) was prepared as a brown solid from 6-(8-trifluoromethylquinolin-4-yloxy)-1-naphthoic acid (38.3 mg, 0.1 mmol) and 6-(aminomethyl)-N-(2-aminophenyl)nicotinamide (29.0 mg, 0.12 mmol) by an analogous procedure to that described in example 16. $^1H$ NMR (DMSO-$d_6$) $\delta$ 4.64 (d, J= 5.6 Hz, 2H, -$CH_2$), 4.87 (s, 2H, benzene-$NH_2$), 6.60 (t, J= 7.2 Hz, 1H, Ar-H), 6.78 (d, J= 7.8 Hz, 1H, Ar-H), 6.89 (d, J= 5.1 Hz, 1H, Ar-H), 6.97 (t, J= 7.2 Hz, 1H, Ar-H), 7.18 (d, J= 7.9 Hz, 1H, Ar-H), 7.53-7.66 (m, 4H, Ar-H), 7.74 (d, J= 6.9 Hz, 1 H, Ar-H), 7.83 (t, J= 7.9 Hz, 1 H, Ar-H), 7.95-8.08 (m, 4H, Ar-H), 8.29 (d, J= 7.0 Hz, 1 H, Ar-H), 8.42 (d, J= 9.1 Hz, 1 H, Ar-H), 8.69 (d, J= 8.3 Hz, 1H, Ar-H), 8.86 (d, J= 5.0 Hz, 1H, Ar-H), 9.22 (t, J= 5.5 Hz, 1H, -CONH), 9.61 (s, 1H, benzene-NH).LC-MS (m/z) 607(M+1).

Example 45

| Preparation of tablets | |
|---|---|
| Formula (1000 tablets): | |
| Compound 31 | 5g |
| Microcrystalline cellulose | 90g |
| Sodium carboxymethyl starch | 5g |
| 4% Polyvidone (K30) solution in absolute ethanol | 50g |
| Talc powder | 0.5g |

Compound 31 was sieved through a 100-mesh sieve. Microcrystalline cellulose, sodium carboxymethyl starch and talc powder were sieved through an 80-mesh sieve respectively. Microcrystalline cellulose and sodium carboxymethyl starch were weighed in formulated amount and blended with compound 31 uniformly in cumulative increment method. Suitable amount of 4% Polyvidone (K30) solution in absolute ethanol was added to produce wet granules. The granules were dried and talc powder in formulated amount was added. Then tablet compression was performed to obtain tablets.

Example 46

| Preparation of capsules | |
|---|---|
| Formula (1000 capsules): | |
| Compound 31 | 5g |
| Microcrystalline cellulose | 55g |
| Lactose | 35g |
| Sodium carboxymethyl starch | 5g |
| Magnesium stearate | 0.5g |

Compound 31 was sieved through a 100-mesh sieve. Microcrystalline cellulose, lactose, sodium carboxymethyl starch and magnesium stearate were sieved through an 80-mesh sieve respectively. Microcrystalline cellulose, lactose and sodium carboxymethyl starch were weighed in formulated amount and blended with compound 31 uniformly in

cumulative increment method. Then magnesium stearate was added in formulated amount and mixed uniformly. Then capsule filling was performed to obtain capsules.

Example 47

Preparation of injection

Formula:

| | |
|---|---|
| Compound 31 | 1.00mg |
| DMSO | 0.10ml |
| Ethanol | 1.00ml |

Compound 31 was dissolved in DMSO, and then ethanol was added to obtain the injection.

Example 48

PDGF and VEGF ligand-dependent cell proliferation assay by compounds of formula (I)

Measurement of *in vivo* inhibition on receptor ligand-dependent cell proliferation:

1. PDGF dependent cell proliferation:

NIH-3T3 mouse fibroblasts cell line engineered to stably express human PDGFRβ was constructed and used to evaluate PDGF dependent cell proliferation. PDGFRβ NIH-3T3 cells were plated into 96-well plates at 5,000 per well and incubated overnight with serum-free medium after 24 hours. Compounds to be tested and PDGF BB (50ng/ml) were added and incubated for 72 hours in serum-free medium. The effects on proliferation were determined by MTS method (Promega) according to the instruction. Incubation was performed for 2 hours at 37°C in $CO_2$ incubator, and absorbance at 490nm was measured using an ELISA plate reader.

2. VEGF dependent cell proliferation:

HUVEC cells were plated into 96-well plates at 6,000 per well and after 24 hours incubated with serum-free medium for 2 hours. Compounds to be tested and VEGF 165 (50ng/ml) were added and incubated for 72 hours in serum-free medium. The effects on cell proliferation were determined by MTS method (Promega) according to the instruction. Incubation was performed for 2 hours at 37°C in $CO_2$ incubator, and absorbance at 490nm was determined using an ELISA plate reader.
The experimental results are shown in Table 2.

Table 2

| Example (compound) | $GI_{50}$ nM (PDGF ligand-dependent cell proliferation) | $GI_{50}$ nM (VEGF ligand-dependent cell proliferation) |
|---|---|---|
| 16 | 48 | 3 |
| 17 | 40 | 3 |
| 18 | 15 | 7 |
| 19 | 11 | 23 |
| 20 | 23 | 6 |
| 21 | 19 | 5 |
| 22 | 372 | 3 |
| 23 | 148 | 18 |
| 25 | 69 | 13 |
| 31 | 46 | 5 |

(continued)

| Example (compound) | $GI_{50}$ nM (PDGF ligand-dependent cell proliferation) | $GI_{50}$ nM (VEGF ligand-dependent cell proliferation) |
|---|---|---|
| 32 | 20 | 2 |
| 33 | 300 | 8 |
| 34 | 248 | 90 |
| 35 | 5 | 1 |
| 36 | 3 | 2 |
| 37 | 159 | 4 |
| 38 | 74 | 25 |
| 39 | 32 | 107 |
| 40 | 1000 | 1000 |
| 41 | 479 | 105 |
| 42 | 48 | 1000 |
| 43 | 1000 | 288 |
| 44 | 1000 | 1000 |

Example 49

*In vitro* inhibition of total HDAC enzyme activity and *in vivo* inhibition of HDAC subtype activity by compounds of formula (I)

Assay of *in vitro* total HDAC enzyme activity:

The *in vitro* total HDAC enzyme activity was determined by HDAC Fluorimetric Assay/Drug Discovery Kit (BIOMOL) according to manufacture's instruction.
The principle of the experiment is as follows: under the action of histone deacetylase (in the experiment, HeLa cell nuclear extract, which was rich in a variety of subtypes of HDAC, was used), an acetyl group is removed from a special substrate *Fluor de Lys*, thus the free amino is exposed. Upon Developer is added, the substrate will produce fluorescence. For the fluorescence, excitation wavelength is 360nm, and emission wavelength is 460nm. More fully the substrate deacetylated, more higher the fluorescence induced. In the absence of inhibitors, the fluorescence value is taken as the control; when inhibitor is existed, fluorescence value induced will be reduced, while in the case no enzyme is existed (corresponding to the completely inhibition of enzyme activity) the fluorescence value is blank. Generally, after suppression the fluorescence values will be between the control and blank. When analysis is made, blank will be used as 0, and the control will be used as 1. Smaller value means higher inhibitory activity.
1. Add Assay buffer, diluted trichostatin A and test inhibitor to appropriate wells of the microtiter plate. Following table lists the amounts used for each regent of various assay types.

| Regent | Assay Buffer | HeLa Extract (Dilution) | Inhibitor (5x) | *Fluor de Lys*™ Substrate (2x) |
|---|---|---|---|---|
| Blank | 25 μl | 0 | 0 | 25 μl |
| Control | 10 μl | 15 μl | 0 | 25 μl |
| Trichostatin A | 0 | 15 μl | 10 μl | 25 μl |
| Test Sample | 0 | 15 μl | 10 μl | 25 μl |

2. Add diluted HeLa nucleoprotein extract to all wells except those that are marked as "blank".
3. Allow diluted *Fluor de Lys*™ Substrate and the samples in the microtiter plate to equilibrate to 25°C.
4. Initiate HDAC reactions by adding diluted substrate (25 μl) to each well and mixing thoroughly.
5. Allow reactions to proceed for 30 minutes and then stop them by addition of *Fluor de Lys*™ Developer (50 μl). Incubate plate at room temperature (25°C) for 10-15 min.

6. Read fluorescence of samples on a microtiter-plate reading fluorimeter at a excitation wavelength of 369 nm and a emitted wavelength of 451 nm.

Assay of selectivity of inhibors on HDAC subtype using reporter gene:

Different HDAC subtypes can bind with different transcriptional factors and involve in expression regulation of various genes. Suitable regulation elements for transcriptional factors are selected to construct reporter genes, which can be used to evaluate the selective inhibition of inhibors on HDAC subtypes. Briefly, HeLa cells were seeded in 96-well plates the day before transfection to give a confluence of 50-80% when transfection. Cells were transfected with one of reporter gene plasmid containing a p21-promoter sequence or response element upstream of a luciferase gene construct using FuGene6 transfection reagent according to the manufacturer's instruction (Roche). For normalizing the transfection efficiency, a GFP expression plasmid was cotransfected. After 24 hours compounds or the vehicle control (DMSO) were added. 24 hours later the cells were harvested and lysed, and the amount of the luciferase was evaluated using luciferase assay kits (Promega) according to the manufacturer's instructions.
The experimental results are shown in Table 3.

Table 3

| Example (compound) | % inhibition of total HDAC enzyme activity at 30μM | Class I HDAC (P21 reporter assay) Fold Induction at 10μM |
|---|---|---|
| CS055 | 50.4 | 33 |
| 16 | 8.6 | 1.3 |
| 17 | 22.5 | 1.1 |
| 18 | 17.1 | 1.1 |
| 19 | 21.9 | 1.4 |
| 20 | 21.9 | 1.5 |
| 21 | 18.6 | 1.1 |
| 22 | 17 | 1.1 |
| 23 | 49.4 | 11.3 |
| 25 | 47.9 | 12.1 |
| 31 | 10.1 | 1.6 |
| 32 | 21.7 | 1.8 |
| 33 | 39.1 | 2.8 |
| 34 | 38.8 | 5.0 |
| 35 | 19.3 | 1.2 |
| 36 | 14.4 | 1.2 |
| 37 | 35.9 | 3.0 |
| 38 | 39.3 | 3.1 |
| 39 | 15.9 | 1.2 |
| 40 | 22.2 | 1.3 |
| 41 | 19.3 | 1.1 |
| 42 | 6.2 | 1.3 |
| 43 | 38.7 | 6.1 |
| 44 | 35.1 | 3.2 |

CS055: Chidamide, a HDAC inhibitor developed by Chipscreen Biosciences, has excellent antitumor activities and is currently in phase II clinical.

Example 50

Inhibition of compounds of formula (I) on tumor cell proliferation

Tumor cells were trypsinized and plated into 96-well plates at 3,000 per well and incubated in complete medium with 10% FBS for 24 hours. Compounds to be tested and vehicle control were added, and the final concentration of compound is in the range of 100 nmol/L to 100 μmol/L. The compounds were incubated for 72 hours in complete medium. The MTS reagent (Promega) was added according to the instruction, incubated for 2 hours at 37°C in $CO_2$ incubator. Then absorbance at 490nm is read using an ELISA plate reader.
The experimental results are shown in Table 4.

Table 4

| Example (compound) | $GI_{50}$ μM in A-498 | $GI_{50}$ μM in A549 | $GI_{50}$ μM in Bel-7402 | $GI_{50}$ μM in HCT-8 | $GI_{50}$ μM in MCF-7 |
|---|---|---|---|---|---|
| CS055 | 12.08 | 11.15 | 18.93 | 7.711 | 3.865 |
| 16 | 30.0 | 30.0 | 30.0 | 12.3 | 30.0 |
| 17 | 30.0 | 30.0 | 30.0 | 3.0 | 30.0 |
| 18 | nd | nd | nd | nd | nd |
| 19 | nd | nd | nd | nd | nd |
| 20 | nd | nd | nd | nd | nd |
| 21 | nd | nd | nd | nd | nd |
| 22 | nd | nd | nd | nd | nd |
| 23 | 14.7 | 30.0 | 30.0 | 5.7 | 4.3 |
| 25 | 14.7 | 30.0 | 30.0 | 4.9 | 6.1 |
| 31 | 9.5 | 17.3 | 30.0 | 6.6 | 10.2 |
| 32 | 7.5 | 8.3 | 17.3 | 6.6 | 15.9 |
| 33 | 1.9 | 2.1 | 2.8 | 1.5 | 2.0 |
| 34 | 7.9 | 11.2 | 17.7 | 5.5 | 5.2 |
| 35 | 9.1 | 7.7 | 19.5 | 8.9 | 13.3 |
| 36 | 4.2 | 7.4 | 12.1 | 4.1 | 8.9 |
| 37 | 30.0 | 30.0 | 30.0 | 30.0 | 30.0 |
| 38 | 6.9 | 30.0 | 30.0 | 8.0 | 9.4 |
| 39 | nd | nd | nd | nd | nd |
| 40 | nd | nd | nd | nd | nd |
| 41 | nd | nd | nd | nd | nd |
| 42 | nd | nd | nd | nd | nd |
| 43 | nd | nd | nd | nd | nd |
| 44 | nd | nd | nd | nd | nd |

nd* : not determined
CS055: Chidamide, a HDAC inhibitor developed by Chipscreen Biosciences, has excellent antitumor activities and is currently in phase II clinical.

Example 51

Inhibition of compound 31 on nude mice tumor transplanted from human A549 lung cancer

Female nu/nu mice of 14~16g were fed by normal diet for 3 days. Then the cultured A549 human lung cancer cells were implanted into the armpit of 50 mice. When tumors had reached more than 6 mm in diameter, the mice were divided into 6 groups randomly. Each group had 8 mice. One group was treated with vehicle. One group was treated with Sutent, the positive control drug. The other four groups were treated with compound 31 at doses 5, 10, 20 and 40 mg/kg body weight. Each group was dosed orally once a day for 24 days. Tumor volumes along with body weights were recorded twice per week. On the next day after 24 doses, the mice were killed, and tumors were weighed. The tumor growth inhibition of each group was calculated using the following formula: {[(the average tumor weight of control group)-(the average tumor weight of test group)]/(the average tumor weight of control group)}×100%.
The experimental results are shown in Table 5 and Figure 1.

Table 5

| Group[a] | Dose (mg/kg) | Body weight (g) | | Tumor weight (g) | TGI (%)[b] | P |
|---|---|---|---|---|---|---|
| | | start | end | | | |
| vehicle | - | 20.3±0.9 | 25.4±2.3 | 4.20±0.75 | - | - |
| Sutent | 40 | 20.3±1.4 | 24.4±2.3 | 2.06±0.71 | 50.9 | <0.001 |
| Compound 31 | 40 | 20.0±0.9 | 22.6±2.4 | 1.06±0.54 | 74.8 | <0.001 |
| Compound 31 | 20 | 20.6±1.1 | 24.2±0.7 | 1.50±0.41 | 64.3 | <0.001 |
| Compound 31 | 10 | 19.9±1.3 | 25.1±1.3 | 2.13±0.51 | 49.4 | <0.001 |
| Compound 31 | 5 | 21.1±0.6 | 24.6±1.3 | 2.20±0.57 | 47.6 | <0.001 |

[a] n = 8 animals per group. [b] Tumor growth inhibition.

Example 52

Inhibition of compound 31 on nude mice tumor transplanted from human HCT-8 colon cancer

Female nu/nu mice of 18~20g were fed by normal diet for 3 days. Then the cultured HCT-8 human colon cancer cells were implanted into the armpit of 50 mice. When tumors had reached a volume of not less than 100 $mm^3$, the mice were divided into 6 groups randomly. Each group had 8 mice. One group was treated with vehicle. One group was treated with Sutent, the positive control drug. The other four groups were treated with compound 31 at doses 2.5, 5, 10 and 20 mg/kg body weight. Each group was dosed orally once a day for 24 days. Tumor volumes along with body weights were recorded twice per week. On the next day after 20 doses, the mice were killed, and tumors were weighed. The tumor growth inhibition of each group was calculated using the following formula:

{[(the average tumor weight of control group)-(the average tumor weight of test group)]/(the average tumor weight of control group)}×100%.

The experimental results are shown in Table 6 and Figure 2.

Table 6

| Group [a] | Dose (mg/kg) | Body weight (g) | | Tumor weight (g) | TGI (%) [b] | P |
|---|---|---|---|---|---|---|
| | | start | end | | | |
| vehicle | - | 20.8±1.0 | 22.1±2.1 | 4.78±1.99 | - | - |
| Sutent | 40 | 21.5±0.7 | 22.4±1.1 | 0.23±0.07 | 95.3 | <0.001 |
| Compound 31 | 20 | 20.5±1.3 | 22.5±1.6 | 0.19±0.06 | 96.1 | <0.001 |

(continued)

| Group [a] | Dose (mg/kg) | Body weight (g) | | Tumor weight (g) | TGI (%) [b] | P |
|---|---|---|---|---|---|---|
| | | start | end | | | |
| Compound 31 | 10 | 20.7±1.1 | 23.7±0.8 | 0.46±0.15 | 90.3 | <0.001 |
| Compound 31 | 5 | 21.6±1.4 | 24.8±1.5 | 0.78±0.25 | 83.8 | <0.001 |
| Compound 31 | 2.5 | 20.3±0.8 | 24.5±1.1 | 2.18±1.28 | 54.5 | <0.001 |
| [a] n = 8 animals per group. [b] Tumor growth inhibition. | | | | | | |

Example 53

Inhibition of compound 31 on nude mice tumor transplanted from human SSMC7721 colon cancer

Female nu/nu mice of 18~20g were fed by normal diet for 3 days. Then the cultured SSMC7721 human liver cancer cells were implanted into the armpit of 50 mice. When tumors had reached a volume of not less than 100 $mm^3$, the mice were divided into 6 groups randomly. Each group had 8 mice. One group was treated with vehicle. One group was treated with Sutent, the positive control drug. The other four groups were treated with compound 31 at doses 2.5, 5, 10 and 20 mg/kg. Each group was dosed orally once a day for 24 days. Tumor volumes along with body weights were recorded twice per week.On the next day after 24 doses, the mice were killed, and tumors were weighed. The tumor growth inhibition of each group was calculated using the following formula:

{[(the average tumor weight of control group)-(the average tumor weight of test group)]/(the average tumor weight of control group)}×100%.

The experimental results are shown in Table 7 and Figure 3.

Table 7

| Group [a] | Dose (mg/kg) | Body weight (g) | | Tumor weight (g) | TGI (%) [b] | P |
|---|---|---|---|---|---|---|
| | | start | end | | | |
| vehicle | - | 20.8±0.8 | 25.1±1.5 | 4.78±1.99 | - | - |
| Sutent | 40 | 21.0±0.8 | 24.8±1.2 | 1.00±0.68 | 70.3 | <0.001 |
| Compound 31 | 20 | 20.2±1.7 | 21.0±2.2 | 0.53±0.28 | 84.4 | <0.001 |
| Compound 31 | 10 | 20.4±1.6 | 23.6±1.5 | 0.70±0.45 | 79.2 | <0.001 |
| Compound 31 | 5 | 20.8±1.2 | 24.8±1.5 | 1.16±0.55 | 65.4 | <0.001 |
| Compound 31 | 2.5 | 20.1±0.9 | 23.2±2.1 | 1.63±0.70 | 51.7 | <0.001 |
| [a] n = 8 animals per group. [b] Tumor growth inhibition. | | | | | | |

Example 54

Inhibition of compound 33 and compound 34 on nude mice tumor transplanted from human HCT-8 colon cancer

Female nu/nu mice of 18~20g were fed by normal diet for 3 days. Then the cultured HCT-8 human colon cancer cells were implanted into the armpit of 50 mice. When tumors had reached a volume of not less than 100 $mm^3$, the mice were divided into 6 groups randomly. Each group had 8 mice. One group was treated with vehicle. One group was treated with Sutent, the positive control drug. Two groups were treated with compound 33 at different concentrations. The other two groups were treated with compound 34 at different concentrations. Each group was dosed orally once a day for 20 days. Tumor volumes along with body weights were recorded twice per week.On the next day after 20 doses, the mice were killed, and tumors were weighed. The tumor growth inhibition of each group was calculated using the following formula:

{[(the average tumor weight of control group)-(the average tumor weight of test group)]/(the average tumor weight of control group)}×100%.

The experimental results are shown in Table 8 and Figure 4.

Table 8

| Group [a] | Dose (mg/kg) | Body weight (g) | | Tumor weight (g) | TGI (%) [b] | P |
|---|---|---|---|---|---|---|
| | | start | end | | | |
| vehicle | - | 19.4±1.6 | 21.2±2.4 | 4.08±0.95 | - | - |
| Sutent | 40 | 20.6±1.2 | 22.1±1.5 | 0.44±0.15 | 89.1 | <0.001 |
| Compound 33 | 60 | 19.4±0.8 | 21.4±1.5 | 1.98±0.61 | 51.5 | <0.001 |
| Compound 33 | 30 | 19.0±1.3 | 21.1±2.2 | 2.31±0.43 | 43.3 | <0.001 |
| Compound 34 | 60 | 19.6±1.1 | 21.6±2.3 | 2.74±0.77 | 32.7 | <0.001 |
| Compound 34 | 30 | 19.7±1.2 | 21.2±1.9 | 3.95±0.73 | 3.07 | >0.05 |

[a] n = 8 animals per group. [b] Tumor growth inhibition.

Example 55

Inhibition of compound 33 and compound 37 on nude mice tumor transplanted from human HCT-8 colon cancer

Female nu/nu mice of 18~20g were fed by normal diet for 3 days. Then the cultured HCT-8 human colon cancer cells were implanted into the armpit of 50 mice. When tumors had reached a vomume of not less than 100 $mm^3$, the mice were divided into 6 groups randomly. Each group had 8 mice. One group was treated with vehicle. One group was treated with Sutent, the positive control drug. Two groups were treated with compound 33 at different concentrations. The other two groups were treated with compound 37 at different concentrations. Compound 33 was administered twice a day with an interval of 6 hours. For other groups, administration was performed once a day. Each group was dosed orally for 20 days. Tumor volumes along with body weights were recorded twice per week.On the next day after 20 doses, the mice were killed, and tumors were weighed. The tumor growth inhibition of each group was calculated using the following formula:

{[(the average tumor weight of control group)-(the average tumor weight of test group)]/(the average tumor weight of control group)}×100%.

The experimental results are shown in Table 9 and Figure 5.

Table 9

| Group [a] | Dose (mg/kg) | Body weight (g) | | Tumor weight (g) | TGI (%) [b] | P |
|---|---|---|---|---|---|---|
| | | start | end | | | |
| vehicle | - | 21.1±0.7 | 23.4±1.5 | 6.13±0.28 | - | - |
| Sutent | 40 | 21.3±0.6 | 23.7±0.8 | 0.29±0.08 | 95.3 | <0.001 |
| Compound 33 | 60x2 | 20.1±0.9 | 19.0±1.8 | 0.45±0.05 | 92.6 | <0.001 |
| Compound 33 | 30x2 | 21.1±1.2 | 22.6±1.6 | 0.73±0.36 | 88.1 | <0.001 |
| Compound 37 | 60 | 20.8±0.8 | 24.1 ±2.1 | 3.36±0.80 | 45.1 | <0.001 |
| Compound 37 | 30 | 20.6±0.8 | 23.6±2.2 | 3.89±1.19 | 36.5 | <0.001 |

[a] n = 8 animals per group. [b] Tumor growth inhibition.

Example 56

Inhibition of compound 33 and compound 37 on nude mice tumor transplanted from human SSMC7721 liver cancer

Female nu/nu mice of 18~20g were fed by normal diet for 3 days. Then the cultured SSMC7721 human liver cancer cells were implanted into the armpit of 50 mice. When tumors had reached to a volume of not less than 100 $mm^3$, the mice were divided into 6 groups randomly. Each group had 8 mice. One group was treated with vehicle. One group was treated with Sutent, the positive control drug. Two groups were treated with compound 33 at different concentrations. The other two groups were treated with compound 37 at different concentrations. Each group was dosed orally once a day for 30 days. Tumor volumes along with body weights were recorded twice per week.On the next day after 30 doses, the mice were killed, and tumors were weighed. The tumor growth inhibition of each group was calculated using the following formula:

{[(the average tumor weight of control group)-(the average tumor weight of test group)]/(the average tumor weight of control group)}×100%.

The experimental results are shown in Table 10 and Figure 6.

Table10

| Group [a] | Dose (mg/kg) | Body weight (g) | | Tumor weight (g) | TGI (%) [b] | P |
|---|---|---|---|---|---|---|
| | | start | end | | | |
| vehicle | - | 21.1±0.4 | 24.5±1.6 | 2.25±0.85 | - | - |
| Sutent | 40 | 21.2±1.1 | 24.0±0.6 | 0.88±0.39 | 61.1 | <0.001 |
| Compound 33 | 60 | 21.4±1.3 | 25.4±2.8 | 1.48±0.89 | 34.4 | >0.05 |
| Compound 33 | 30 | 20.8±0.5 | 24.0±1.7 | 1.63±0.47 | 27.8 | >0.05 |
| Compound 37 | 60 | 21.4±0.6 | 24.3±1.1 | 1.28±0.51 | 43.3 | <0.05 |
| Compound 37 | 30 | 20.7±1.2 | 25.3±0.9 | 1.45±0.58 | 35.6 | <0.05 |

[a] n = 8 animals per group. [b] Tumor growth inhibition.

## Claims

**1.** A compound of formula I:

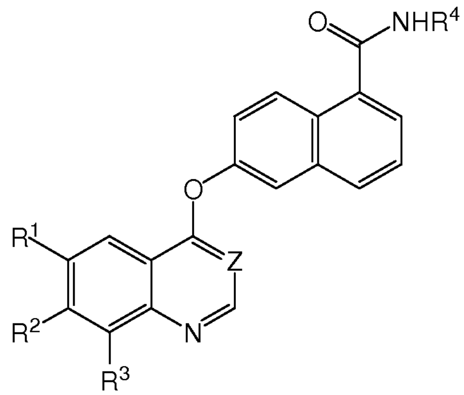

( I )

including its free form, salt form, stereoisomer, enantiomer, diastereomer, or hydrate, wherein

Z is CH or N;
$R^1$, $R^2$ and $R^3$ are each hydrogen, halo, alkyl, alkoxy or trifluoromethyl;
$R^4$ is

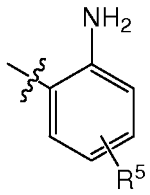 , or 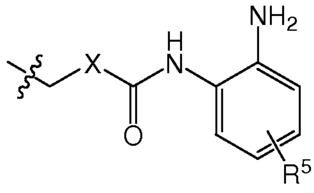 ;

X is a benzene ring or a pyridine ring;
$R^5$ is one or more substituents selected from the group consisting of hydrogen, halo, alkyl, alkoxy and trifluoromethyl.

2. The compound of claim 1, wherein
Z is CH;
$R^1$, $R^2$ and $R^3$ are each hydrogen, halo, alkyl, alkoxy or trifluoromethyl;
$R^4$ is

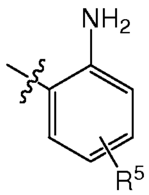 , or 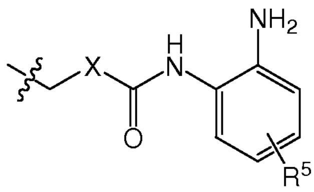 ;

X is a benzene ring or a pyridine ring;
$R^5$ is one or more substituents selected from the group consisting of hydrogen, halo, alkyl, alkoxy and trifluoromethyl.

3. The compound of claim 1, wherein
Z is CH;
$R^1$, $R^2$ and $R^3$ are each hydrogen or alkoxy;
$R^4$ is

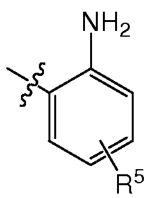 , or 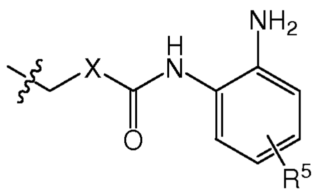 ;

X is a benzene ring or a pyridine ring;
$R^5$ is one or more substituents selected from the group consisting of hydrogen, halo, alkyl, alkoxy and trifluoromethyl.

4. The compound of claim 1, wherein
Z is CH;
$R^1$ and $R^2$ are each hydrogen or methoxy;
$R^3$ is H;
$R^4$ is

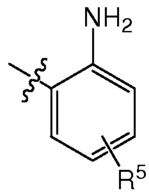
, or
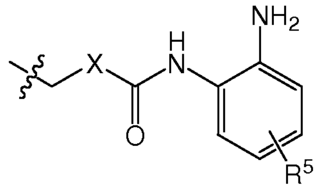
;

X is a benzene ring or a pyridine ring;
$R^5$ is one or more substituents selected from the group consisting of hydrogen, halo, alkyl, alkoxy and trifluoromethyl.

**5.** The compound of claim 1, wherein
Z is CH;
$R^1$ and $R^2$ are each hydrogen or methoxy;
$R^3$ is H;
$R^4$ is

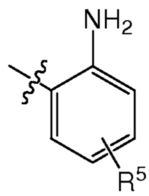
, or
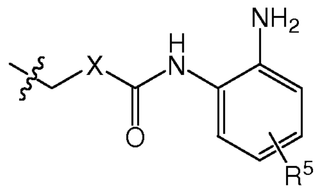
;

X is a benzene ring or a pyridine ring;
$R^5$ is H or F.

**6.** A method for preparing a compound of formula I

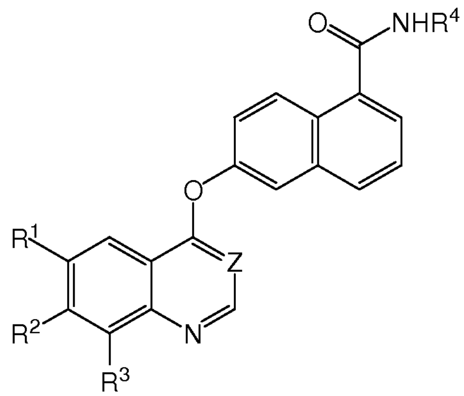

**(I)**

wherein
Z is CH or N;
$R^1$, $R^2$ and $R^3$ are each hydrogen, halo, alkyl, alkoxy or trifluoromethyl; $R^4$ is

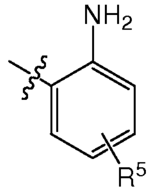 , or 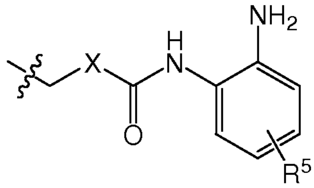 ;

X is a benzene ring or a pyridine ring;
$R^5$ is one or more substituents selected from the group consisting of hydrogen, halo, alkyl, alkoxy and trifluoromethyl;
comprising reacting
a compound of formula (**II**)

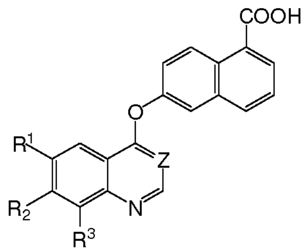

(**II**)

with a compound of formula (**III**)

$H_2N\text{-}R^4$ (**III**)

in the presence of an organic solvent and a peptide condensing agent, to form a compound of formula (**I**).

**7.** The method of claim 6, wherein said peptide condensing agent is selected from the group consisting of 1-ethyl-3-(3-dimethyl-aminopropyl)carbodiimide (EDC), N,N'-dicyclohexylcarbodiimide (DCC) and N,N'-carbonyldiimidazole (CDI).

**8.** The method of claim 6, wherein said organic solvent is selected from the group consisting of benzene, toluene, tetrahydrofuran, 1,4-dioxane, dichloromethane, chloroform and N, N-dimethylformamide.

**9.** A pharmaceutical preparation for use in the treatment of diseases associated with abnormal protein kinase activities or abnormal histone deacetylase activities comprising a compound of formula (I) according to claim 1 and pharmaceutically acceptable carrier, excipient, or diluent.

**10.** The pharmaceutical preparation as claimed in claim 9, in the form of a tablet, capsule, powder, syrup, solution, suspension, injection or ointment.

**11.** The compound of claim 1 for use in the treatment of inflammatory diseases, autoimmune diseases, cancer, nervous system diseases and neurodegenerative diseases, allergies, asthma, cardiovascular diseases and metabolic diseases, or hormone-related diseases.

**12.** The pharmaceutical preparation of claim 9 for use in the treatment of inflammatory diseases, autoimmune diseases, cancer, nervous system diseases and neurodegenerative diseases, allergies, asthma, cardiovascular diseases and metabolic diseases, or hormone-related diseases.

**13.** The pharmaceutical preparation of claim 9, comprising an amount within the range of 0.001 to 200 mg of said compound of formula (I).

**Patentansprüche**

1. Verbindung der Formel I:

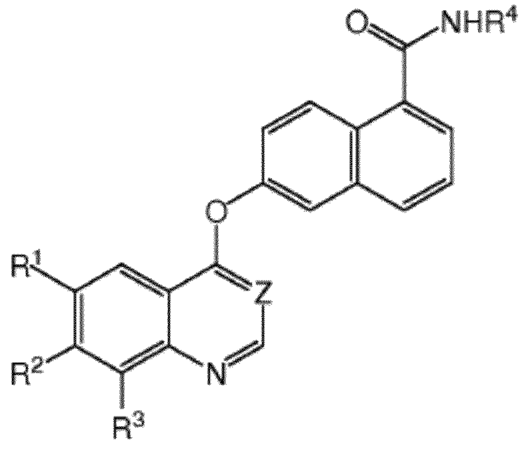

(I)

einschließlich einer freien Form, einer Salzform, eines Stereoisomers, eines Enantiomers, eines Diastereomers oder eines Hydrats davon,
wobei

Z CH oder N ist;
$R^1$, $R^2$ und $R^3$ jeweils Wasserstoff, Halo, Alkyl, Alkoxy oder Trifluormethyl sind;
$R^4$

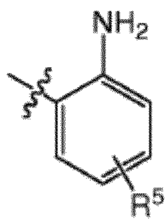

oder

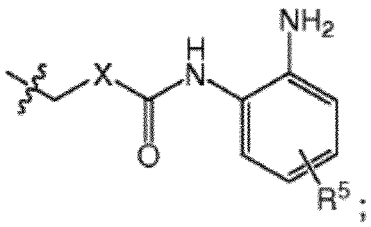

ist,
X ein Benzolring oder Pyridinring ist;
$R^5$ ein oder mehrere Substituenten ist, die aus der Gruppe ausgewählt sind, bestehend aus Wasserstoff, Halo, Alkyl, Alkoxy und Trifluormethyl.

2. Verbindung nach Anspruch 1, wobei
Z CH ist;
$R^1$, $R^2$ und $R^3$ jeweils Wasserstoff, Halo, Alkyl, Alkoxy oder Trifluormethyl sind; $R^4$

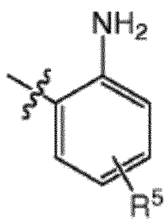

oder

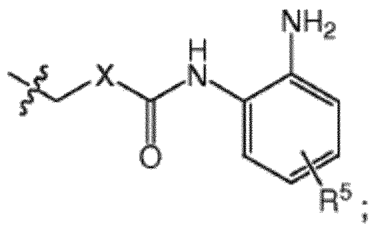

ist;
X ein Benzolring oder ein Pyridinring ist;
$R^5$ ein oder mehrere Substituenten ist, die aus der Gruppe ausgewählt sind, bestehend aus Wasserstoff, Halo, Alkyl, Alkoxy und Trifluormethyl.

**3.** Verbindung nach Anspruch 1, wobei
Z CH ist;
$R^1$, $R^2$ und $R^3$ jeweils Wasserstoff oder Alkoxy sind;
$R^4$

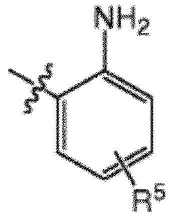
oder
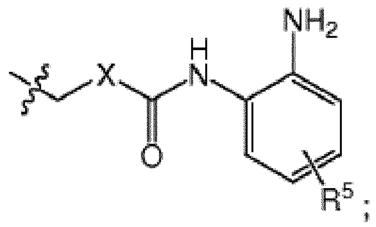

ist;
X ein Benzolring oder ein Pyridinring ist;
$R^5$ ein oder mehrere Substituenten ist, die aus der Gruppe ausgewählt sind, bestehend aus Wasserstoff, Halo, Alkyl, Alkoxy und Trifluormethyl.

**4.** Verbindung nach Anspruch 1, wobei
Z CH ist;
$R^1$ und $R^2$ jeweils Wasserstoff oder Methoxy sind;
$R^3$ H ist;
$R^4$

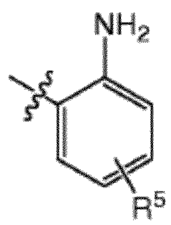
oder
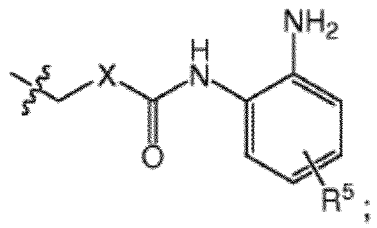

ist;
X ein Benzolring oder ein Pyridinring ist;
$R^5$ ein oder mehrere Substituenten ist, die aus der Gruppe ausgewählt sind, bestehend aus Wasserstoff, Halo, Alkyl, Alkoxy und Trifluormethyl.

**5.** Verbindung nach Anspruch 1, wobei
Z CH ist;
$R^1$ und $R^2$ jeweils Wasserstoff oder Methoxy sind;
$R^3$ H ist;
$R^4$

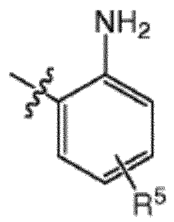

oder

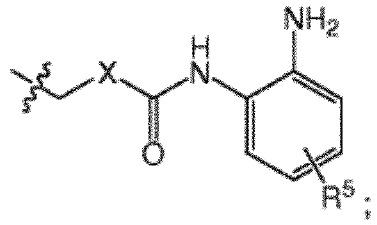

ist;
X ein Benzolring oder ein Pyridinring ist;
$R^5$ H oder F ist.

**6.** Verfahren zum Herstellen einer Verbindung der Formel I

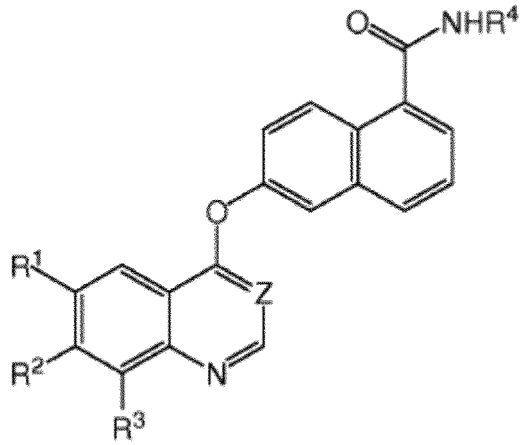

**(I)**

wobei
Z CH oder N ist;
$R^1$, $R^2$ und $R^3$ jeweils Wasserstoff, Halo, Alkyl, Alkoxy oder Trifluormethyl sind; $R^4$

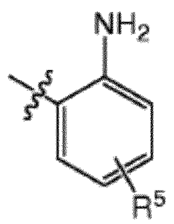

oder

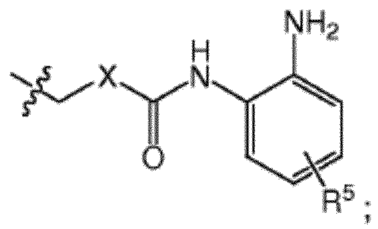

ist,
X ein Benzolring oder Pyridinring ist;
$R^5$ ein oder mehrere Substituenten ist, die aus der Gruppe ausgewählt sind, bestehend aus Wasserstoff, Halo, Alkyl, Alkoxy und Trifluormethyl;
umfassend das Reagieren
einer Verbindung der Formel (II)

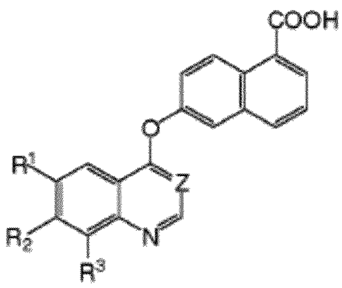

(II)

mit einer Verbindung der Formel (III)

$H_2N-R^4$

in der Gegenwart eines organischen Lösungsmittels und eines Peptidkondensationsmittels, um eine Verbindung der Formel (I) zu bilden.

**7.** Verfahren nach Anspruch 6, wobei das Peptidkondensationsmittel aus der Gruppe ausgewählt ist, bestehend aus 1-Ethyl-3-(3-dimethyl-aminopropyl)carbodiimid (EDC), N,N'-Dicyciohexyicarbodiimid (DCC) und N,N'-Carbonyldiimidazol (CDI).

**8.** Verfahren nach Anspruch 6, wobei das organische Lösungsmittel aus der Gruppe ausgewählt ist, bestehend aus Benzol, Toluol, Tetrahydrofuran, 1,4-Dioxan, Dichlormethan, Chloroform und N,N-Dimethylformamid.

**9.** Pharmazeutische Zubereitung zur Verwendung bei der Behandlung von Krankheiten, die mit anomalen Proteinkinaseaktivitäten oder anomalen Histondeacetylaseaktivitäten assoziiert sind, die eine Verbindung der Formel (I) nach Anspruch 1 und einen pharmazeutisch verträglichen Träger, einen pharmazeutisch verträglichen Exzipienten oder ein pharmazeutisch verträgliches Verdünnungsmittel umfasst.

**10.** Pharmazeutische Zubereitung nach Anspruch 9 in Form einer Tablette, einer Kapsel, eines Pulvers, eines Sirups, einer Lösung, einer Suspension, einer Injektion oder einer Salbe.

**11.** Verbindung nach Anspruch 1 zur Verwendung bei der Behandlung von Entzündungskrankheiten, Autoimmunkrankheiten, Krebs, Erkrankungen des Nervensystems und neurodegenerativen Krankheiten, Allergien, Asthma, Herz-Kreislauf-Krankheiten und Stoffwechselkrankheiten oder hormonbedingten Krankheiten.

**12.** Pharmazeutische Zubereitung nach Anspruch 9 zur Verwendung bei der Behandlung von Entzündungskrankheiten, Autoimmunkrankheiten, Krebs, Erkrankungen des Nervensystems und neurodegenerativen Krankheiten, Allergien, Asthma, Herz-Kreislauf-Krankheiten und Stoffwechselkrankheiten oder hormonbedingten Krankheiten.

**13.** Pharmazeutische Zubereitung nach Anspruch 9, die eine Menge der Verbindung der Formel (I) im Bereich von 0,001 bis 200 mg umfasst.

## Revendications

**1.** Composé de formule I :

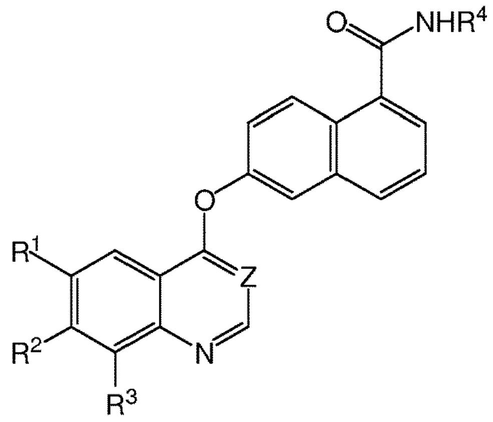

( I )

y compris sa forme libre, sa forme sel, son stéréoisomère, son énantiomère, son diastéréomère, ou son hydrate, formule dans laquelle

Z est CH ou N ;
chacun de $R^1$, $R^2$ et $R^3$ est l'hydrogène, un halogène, ou un radical alkyle, alcoxy ou trifluorométhyle ;
$R^4$ est

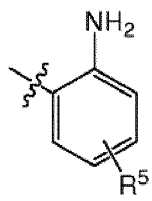

ou

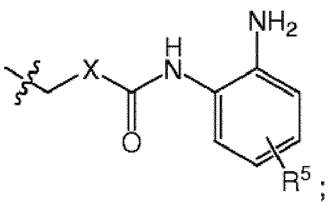

X est un cycle benzène ou un cycle pyridine ;
$R^5$ est un ou plusieurs substituants choisis dans l'ensemble constitué par l'hydrogène, les halogènes, et les radicaux alkyle, alcoxy et trifluorométhyle.

**2.** Composé selon la revendication 1, dans lequel
Z est CH ;
chacun de $R^1$, $R^2$ et $R^3$ est l'hydrogène, un halogène, ou un radical alkyle, alcoxy ou trifluorométhyle ;
$R^4$ est

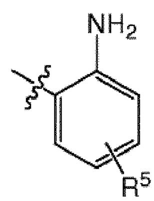

ou

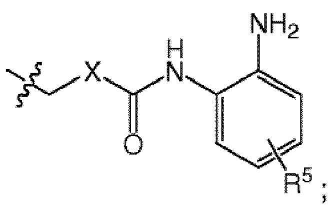

X est un cycle benzène ou un cycle pyridine ;
$R^5$ est un ou plusieurs substituants choisis dans l'ensemble constitué par l'hydrogène, les halogènes, et les radicaux alkyle, alcoxy et trifluorométhyle.

**3.** Composé selon la revendication 1, dans lequel
Z est CH ;
chacun de $R^1$, $R^2$ et $R^3$ est l'hydrogène ou un radical alcoxy ;
$R^4$ est

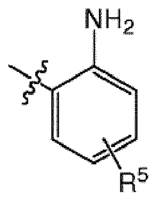

ou

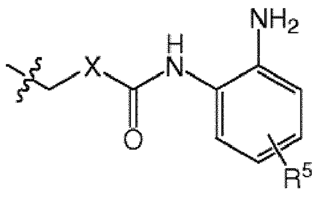

;

X est un cycle benzène ou un cycle pyridine ;
$R^5$ est un ou plusieurs substituants choisis dans l'ensemble constitué par l'hydrogène, les halogènes, et les radicaux alkyle, alcoxy et trifluorométhyle.

**4.** Composé selon la revendication 1, dans lequel
Z est CH ;
chacun de $R^1$ et $R^2$ est l'hydrogène ou un radical méthoxy ;
$R^3$ est H ;
$R^4$ est

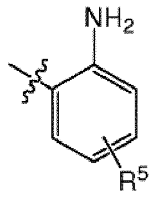

ou

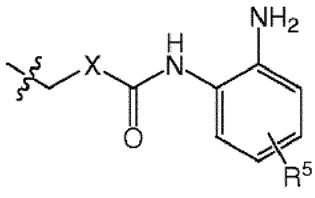

;

X est un cycle benzène ou un cycle pyridine ;
$R^5$ est un ou plusieurs substituants choisis dans l'ensemble constitué par l'hydrogène, les halogènes, et les radicaux alkyle, alcoxy et trifluorométhyle.

**5.** Composé selon la revendication 1, dans lequel Z est CH ;
chacun de $R^1$ et $R^2$ est l'hydrogène ou un radical méthoxy ; $R^3$ est H ;
$R^4$ est

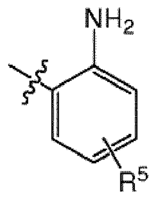

ou

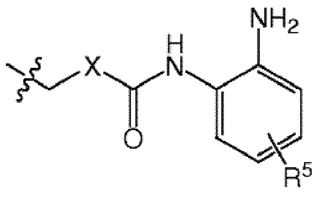

;

X est un cycle benzène ou un cycle pyridine ;
$R^5$ est H ou F.

**6.** Procédé pour préparer un composé de formule 1

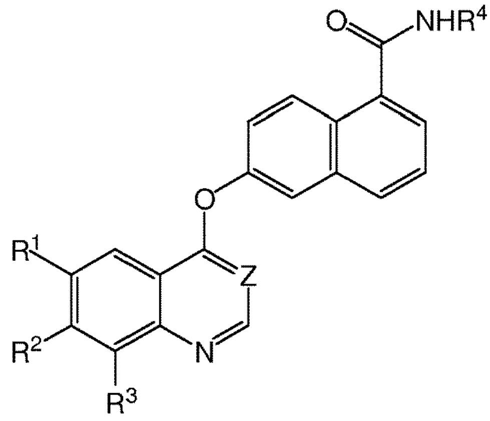

( I )

dans laquelle
Z est CH ou N ;
chacun de $R^1$, $R^2$ et $R^3$ est l'hydrogène, un halogène, ou un radical alkyle, alcoxy ou trifluorométhyle ;
$R^4$ est

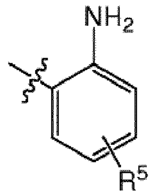 ou 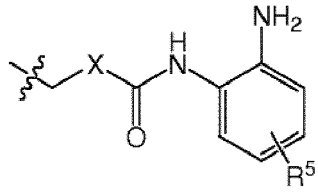 ;

X est un cycle benzène ou un cycle pyridine ;
$R^5$ est un ou plusieurs substituants choisis dans l'ensemble constitué par l'hydrogène, les halogènes, et les radicaux alkyle, alcoxy et trifluorométhyle ;
comprenant la réaction
d'un composé de formule (II)

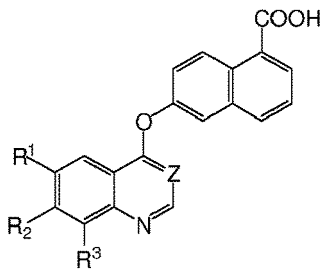

(II)

avec un composé de formule (III)

$H_2N\text{-}R^4$ (III)

en présence d'un solvant organique et d'un agent de condensation peptidique, pour former un composé de formule (I).

**7.** Procédé selon la revendication 6, dans lequel ledit agent de condensation peptidique est choisi dans l'ensemble constitué par le 1-éthyl-3-(3-diméthylamino-propyl)carbodiimide (EDC), le N,N'-dicyclohexylcarbodiimide (DCC) et le N,N'-carbonyldiimidazole (CDI).

**8.** Procédé selon la revendication 6, dans lequel ledit solvant organique est choisi dans l'ensemble constitué par le benzène, le toluène, le tétrahydrofurane, le 1,4-dioxane, le dichlorométhane, le chloroforme et le N,N-diméthylformamide.

**9.** Préparation pharmaceutique pour utilisation dans le traitement de maladies associées à des activités anormales de protéine kinase ou à des activités anormales d'histone désacétylase, comprenant un composé de formule (I) selon la revendication 1 et un véhicule, excipient ou diluant pharmaceutiquement acceptable.

**10.** Préparation pharmaceutique selon la revendication 9, sous la forme d'un comprimé, d'une capsule, d'une poudre, d'un sirop, d'une solution" d'une suspension, d'une composition injectable ou d'une pommade.

**11.** Composé selon la revendication 1 pour utilisation dans le traitement de maladies inflammatoires, de maladies auto-immunes, d'un cancer, de maladies du système nerveux et de maladies neurodégénératives, d'allergies, de l'asthme, de maladies cardiovasculaires et de maladies métaboliques, ou de maladies hormonales.

**12.** Préparation pharmaceutique selon la revendication 9 pour utilisation dans le traitement de maladies inflammatoires, de maladies auto-immunes, d'un cancer, de maladies du système nerveux et de maladies neurodégénératives, d'allergies, de l'asthme, de maladies cardiovasculaires et de maladies métaboliques, ou de maladies hormonales.

**13.** Préparation pharmaceutique selon la revendication 9, comprenant une quantité dudit composé de formule (I) située dans la plage allant de 0,001 à 200 mg.

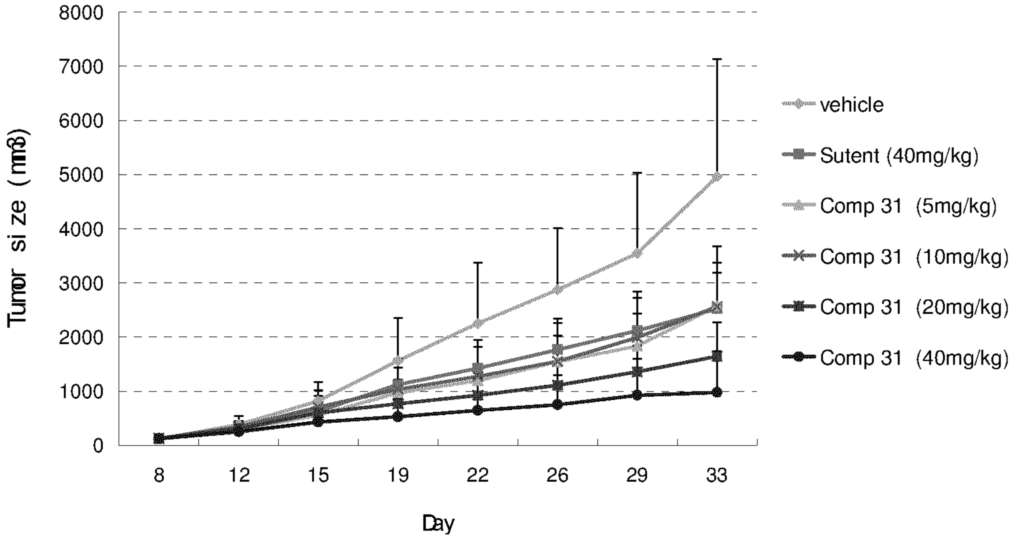


Figure 1

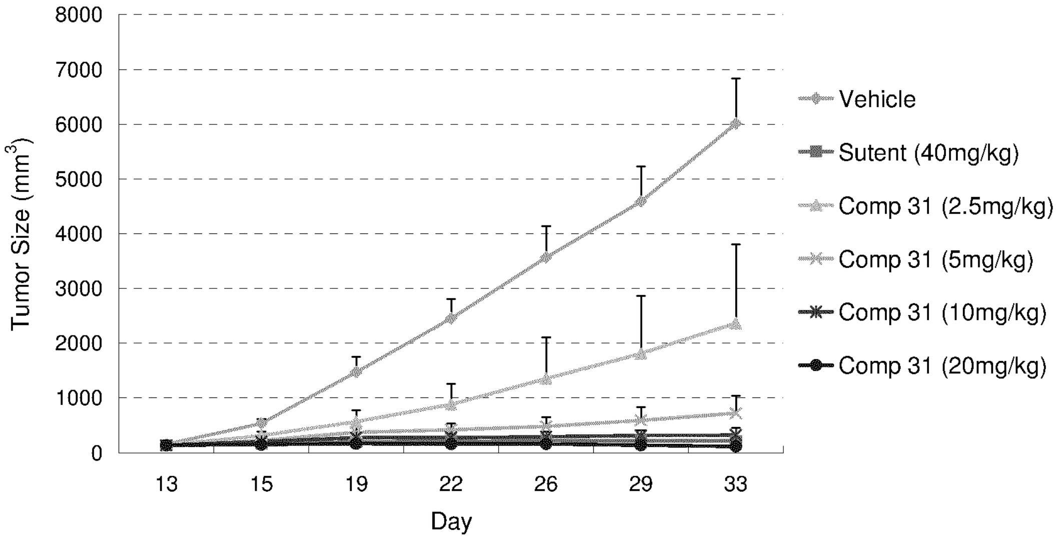


Figure 2

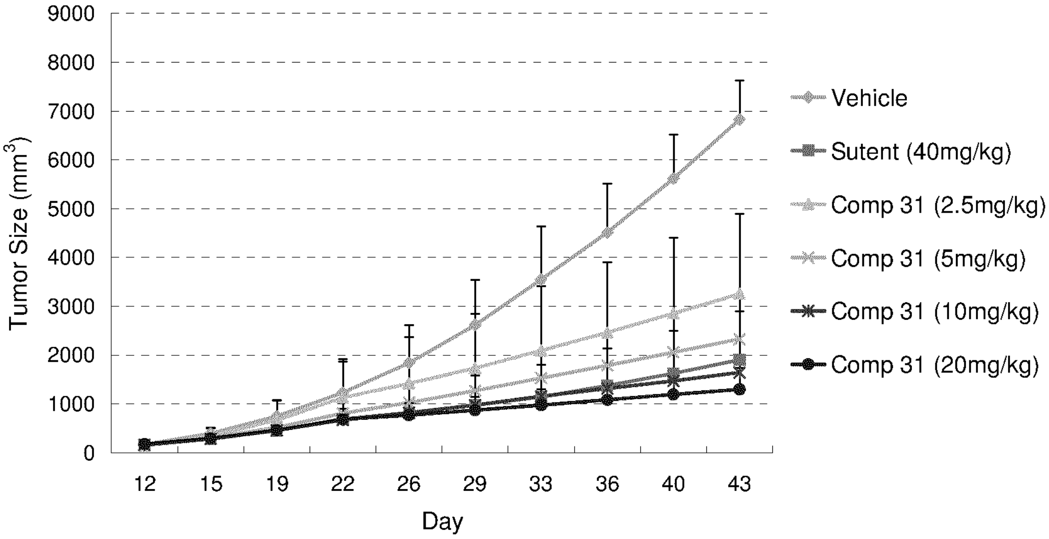


Figure 3

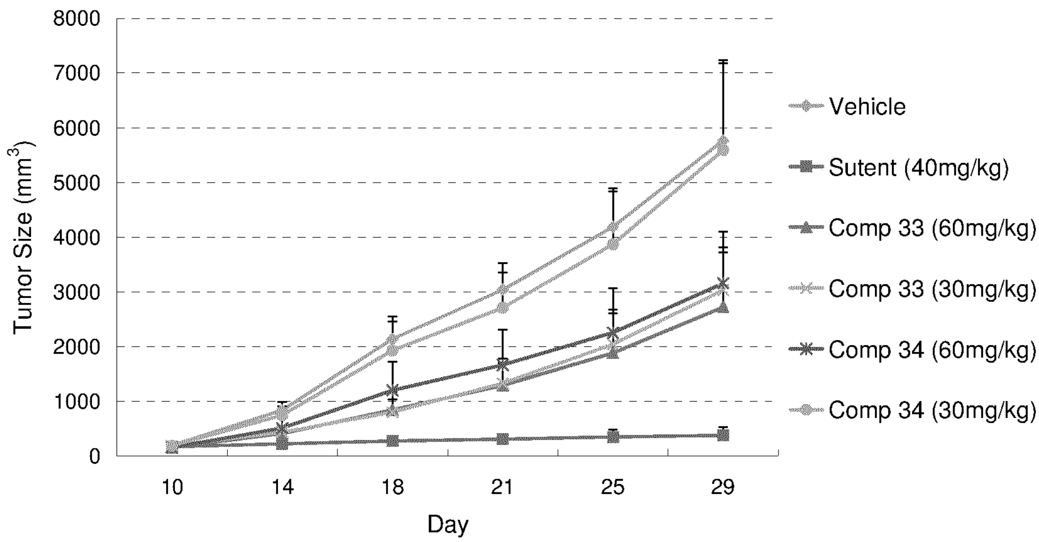


Figure 4

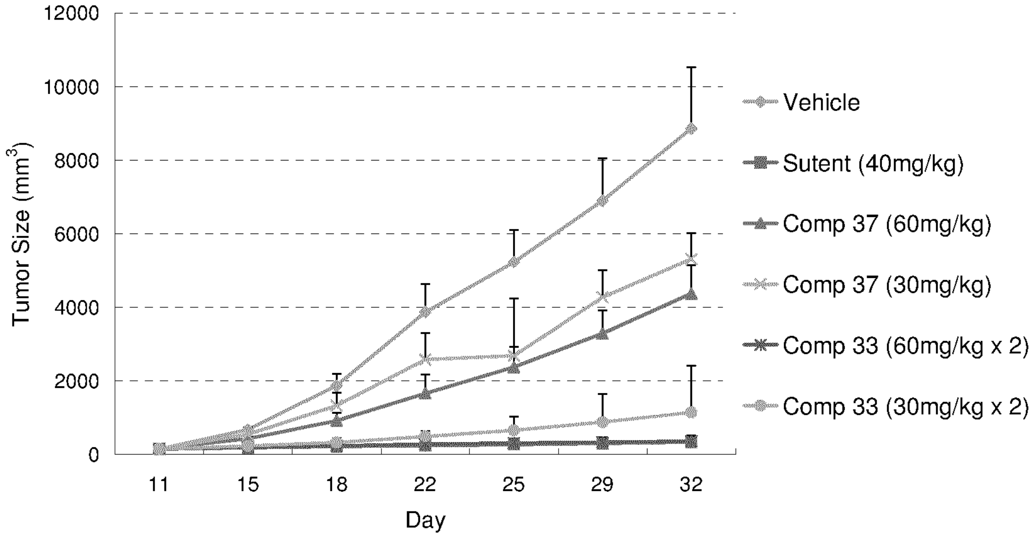


Figure 5

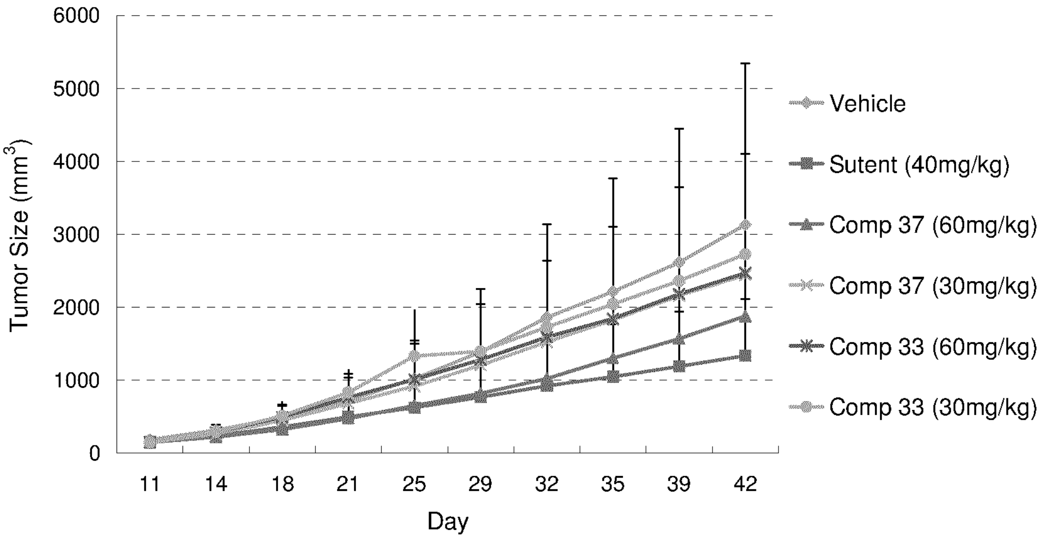


Figure 6

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 7151096 B
- US 7189721 B
- US 7132533 B
- US 7214700 B
- US 7179910 B
- US 7166597 B
- US 7157476 B
- US 7125905 B
- US 7115739 B
- US 7098330 B
- US 7041687 B
- CN 1933839 A
- WO 2005070891 A
- WO 2005021553 A1
- EP 0847992 A1
- US 20020103192 A1
- WO 0226696 A1
- WO 0170675 A2
- WO 0118171 A2


### Non-patent literature cited in the description


- **TAN, S-L.** *J. Immunol,* 2006, vol. 176, 2872-2879
- **HEALY, A.** *J. Immunol,* 2006, vol. 177, 1886-1893
- **SALEK-ARDAKANI, S. et al.** *J. Immunol,* 2005, vol. 175, 7635-7641
- **KIM, J. et al.** *J. Clin. Invest.,* 2004, vol. 114, 823-827
- **ARTEAGA, C-L.** *Curr. Opin. Oncol.,* 2001, vol. 6, 491-498
- **PETIT, A-M. et al.** *Am. J. Pathol.,* 1997, vol. 151, 1523-1530
- **WIKSTRAND, C-J. et al.** *J Natl Cancer Inst.,* 1998, vol. 90, 799-800
- **MENDELSOHN, J.** *Clin. Can. Res.,* 1997, vol. 3, 2707-2707
- **PARIKH, A-A.** *Hematol. Oncol. Clin. N. Am.,* 2004, vol. 18, 951-971
- **HAQ et al.** *J. Cell Biol.,* 2000, vol. 151, 117
- **SCHUMACHER et al.** *J. Cell Biol,* 1998, vol. 143, 1635-1646
- **KIMURA et al.** *J. Biol. Chem,* 1997, vol. 272, 13766-13771
- **MONIA et al.** *Nat. Med.,* 1996, vol. 2, 668-675
- **GRUNSTEIN, M.** *Nature,* 1997, vol. 389, 349-352
- **RUIJTER, A-J-M.** *Biochem. J.,* 2003, vol. 370, 737-749
- **GRIGNANI, F.** *Nature,* 1998, vol. 391, 815-818
- **MARKS, P-A.** *Nature Reviews Cancer,* 2001, vol. 1, 194
- **LANGLEY B et al.** *Current Drug Targets-CNS & Neurological Disorders,* 2005, vol. 4, 41-50
- **DOKMANOVIC, M.** *J. Cell Biochenm.,* 2005, vol. 96, 293-304
- **GLASER, K-B.** *Biochem. Biophys. Res. Comm.,* 2003, vol. 310, 529-536
- **LAGGER, G.** *EMBO J.,* 2002, vol. 21, 2672-2681
- **BARTL. S.** *Mol. Cell Biol.,* 1997, vol. 17, 5033-5043
- **TRIVEDI, C-M.** *Nat. Med,* vol. 13, 324-331
- **WILSON, A-J.** *J. Biol. Chem,* 2006, vol. 281, 13548-13558
- **SAKUMA, T.** *Int. J. Oncol.,* 2006, vol. 29, 117-124
- **GLAROS S et al.** *Oncogene,* 04 June 2007
- **MAI, A et al.** *Int J. Biochem Cell Bio.,* 04 April 2007
- **VINCENT A. et al.** *Oncogene,* 30 April 2007
- **HERMAN D et al.** *Nature Chemical Biology,* 2006, vol. 2 (10), 551-8
- **AVILA AM et al.** *J Clinic Investigation,* 2007, vol. 117 (3), 659-71
- **DE BORE J.** *Tissue Eng.,* 2006, vol. 12 (10), 2927-37
- **GIALITAKIS M et al.** *Nucleic Acids Res.,* 2007, vol. 34 (1), 765-72
- Handbook of Pharmaceutical Excipients. American Pharmaceutical Association, October 1986